# EUROPEAN PATENT SPECIFICATION

(11) **EP 3 585 332 B1**
(45) Date of publication and mention of the grant of the patent: **19.07.2023**
(21) Application number: 18713512.4
(22) Date of filing: 08.03.2018
(51) Int. Cl.: A61F 9/008, B23K 26/0622, B23K 26/08, B23K 26/53, B23K 26/067, B23K 26/082, B23K 103/00, B23K 26/064

(54) **BEAM MULTIPLEXER FOR WRITING REFRACTIVE INDEX CHANGES IN OPTICAL MATERIALS**
STRAHLMULTIPLEXER ZUM SCHREIBEN VON BRECHUNGSINDEXÄNDERUNGEN IN OPTISCHEN MATERIALIEN
MULTIPLEXEUR DE FAISCEAU DESTINÉ À ÉCRIRE DES CHANGEMENTS D'INDICE DE RÉFRACTION DANS DES MATÉRIAUX OPTIQUES

(30) Priority: 31.03.2017 US 201762479826 P
(43) Date of publication of application: 01.01.2020
(73) Proprietor: University of Rochester, Rochester, NY 14642 (US)
(72) Inventor: KNOX, Wayne H., Pittsford, New York 14534 (US)
(74) Representative: Flügel Preissner Schober Seidel
(86) International application number: PCT/US2018/021601
(87) International publication number: WO 2018/182946

(56) References cited:
- WO-A1-2009/003107
- WO-A2-2006/074469
- WO-A2-2009/114375
- JP-A- 2006 068 762
- US-A1- 2007 051 706
- US-A1- 2014 135 920

## Description

### TECHNICAL FIELD

The application relates to using a pulsed laser to modify the refractive index of an optical medium, and particularly to writing refractive index changes into ocular tissues or replacement or augmentative structures to modify or enhance the visual performance of patients.

### BACKGROUND OF THE INVENTION

Pulsed lasers operating within specified regimes specially adapted to target optical materials have been demonstrated to produce localized refractive index changes in the optical materials without otherwise damaging the materials in ways that would impair vision. The energy regimes, while above the nonlinear absorption threshold, are typically just below the breakdown thresholds of the optical materials at which significant light scattering or absorption degrades their intended performance. The considerations of these adapted energy regimes include pulse wavelength, pulse energy, pulse duration, the size and shape into which the pulses are focused into the optical material, and the temporal and physical spacing of the pulses.

Examples include US Patent Application Publication No. 2013/0226162 entitled Method for Modifying the Refractive Index of Ocular Tissues, which discloses a laser system for changing the index of refraction of cornea tissue in a living eye for forming of modifying optical elements including Bragg gratings, microlens arrays, zone plates, Fresnel lenses, and combinations thereof. Here wavelengths are preferably between 400 nm and 900 nm, pulse energies are preferably between 0.01 nJ and 10 nJ, pulse duration is preferably between 10 fs and 100 fs, the repetition rate is preferably between 10 MHz and 500 MHz, the numerical aperture is preferably about 0.70 producing about a line width between approximately 0.6 µm to 1.5 µm and a line depth between 0.4 µm to 8 µm, and the scan rate is between approximately 0.1 µm/s to 10 mm/s. US Patent Application Publication No. 2013/0268072 entitled Optical Hydrogel Material with Photosensitizer and Method for Modifying the Refractive Index discloses a method for modifying the refractive index of an optical, hydrogel polymeric material prepared with a photosensitizer particularly for the purposes of enhancing the efficiency of nonlinear absorption and increasing the scan rate at which refractive structure can be formed. Wavelengths are preferably between 650 nm to 950 nm, pulse energies are preferably between 0.05 nJ to 10 nJ, pulse duration is preferably between 4 fs and 100 fs, the repetition rate includes by way of example both 80 MHz and 93 MHz, the numerical aperture is preferably about 0.70 producing about a line width between approximately 0.6 µm to 10.5 µm and a line depth between 1 µm to 4 µm, and the scan rate is between approximately 0.1 µm/s to 4 mm/s. US Patent Application Publication No. 2015/0126979 entitled Method for Modifying the Refractive Index of an Optical Material discloses the writing of selected regions of optical hydrogel materials prepared with a hydrophilic monomer following implantation of the prepared material into the eye of the patient. Wavelengths are preferably between 600 nm to 900 nm, pulse energies are preferably between 0.01 nJ to 50 nJ, pulse duration is preferably between 4 fs and 100 fs, the repetition rate includes by way of example 93 MHz, the numerical aperture is preferably about 0.70 producing about a line width between approximately 0.2 µm to 3 µm and a line depth between 0.4 µm to 8 µm, and a demonstrated scan rate is approximately 0.4 µm/s. US Patent Application Publication No. 2015/0378065 entitled Method for Modifying the Refractive Index of an Optical Material and resulting Optical Vision Component, which discloses the writing of GRIN layers in optical polymeric materials.. Wavelengths are preferably between 750 nm to 1100 nm, pulse energies are preferably between 0.01 nJ to 20 nJ, pulse duration is preferably between 10 fs and 500 fs, the repetition rate is preferably between 10 MHz and 300 MHz, the numerical aperture is preferably about 0.70 producing about a line width between approximately 0.6 µm to 3 µm and a line depth between 0.4 µm to 8 µm, and the scan rate is between approximately 0.1 mm/s to 10 mm/s.

While more opportunities exist for synergies between the adapted energy regimes and man-made optical materials, the speed and efficiency with which refractive index changes can be written into the optical materials remains of importance whether the optical materials are of living origin or man-made, and whether the optical materials are positioned in vivo or in vitro. Constraints relating to the need to deliver concentrated pulse energies of a laser beam in a form that achieves the desired refractive index changes in the optical materials without exceeding the damage threshold at which the desired optical performance is degraded have limited the speed and efficiency with which refractive index structures can be written into the optical materials.

A method for changing a refractive index of tissue in a living eye is disclosed by WO 2009/003107 A1. The method comprising:
(a) providing a laser capable of emitting laser pulses at an intensity high enough to change the refractive index of a focus spot in the tissue in the living eye, but not high enough to destroy the tissue in the living eye or to affect tissues in the eye outside of the focus spot;
(b) directing said pulses at the tissue of the living eye and focusing said pulses to form the focus spot; and
(c) changing the refractive index of the tissue at locations defined by the focus spot, using the pulses.

WO 2009/114375 A2 discloses a laser-based system for modification of a transparent material. The system comprises a pulsed laser apparatus generating a pulsed laser output; a multifocus beam generator receiving the output. The generator is configured to form a plurality of focused beams using the output, each focused beam having a beam waist, wherein the beam waists are spaced depthwise relative to the material. At least one beam waist of the plurality of focused beams is within the material and causing modification of the material. A motion system serves to produce relative movement between the material and the focused beams. A controller is coupled to the pulsed laser apparatus and to the motion system, and serves to control the system in such a way that the plurality of focused beams are formed during the relative motion.

A method for scribing transparent materials which uses ultrashort laser pulses to create multiple scribe features with a single pass of the laser beam across the material is disclosed by US 2007/051706 A1.

JP 2006 068762 A discloses a method and an apparatus of laser beam machining.

A system which allows hydrophilicity alteration of a polymeric material is disclosed by US 2014/135920 A1.

WO 2006/074469 A2 discloses a system and a method which focus light at various focal points which are at various depths within an eye tissue.

### SUMMARY

The disclosed device incorporates a beam multiplexer for dividing a pulsed laser beam into two or more laser beams whose pulses can be temporally and spatially related to expand opportunities for improving the speed and efficiency with which refractive index structures can be written into a variety of optical materials. The opportunities include increasing the speed at which regions are scanned in the optical materials, achieving greater refractive index changes along the scanned regions, improving continuity or control over the refractive index changes along or between regions, expanding the width or thickness over with the refractive index changes are made along the scanned regions, and scanning over multiple regions within or between designated layers of the optical materials. The pulses of the multiplexed beams can be temporally offset to increase the effective repetition rate at which pulses are delivered to the optical materials and spatially offset to spread the pulses throughout a greater volume for increasing the size of a common volume subject to refractive index change along the same scanned region or subjecting different volumes to the same or different refractive index changes along multiple scanned regions. The temporal and spatial relationships among the different beam pulses delivered to the optical material can also be adapted to affect the sizes and shapes of temperature profiles generated along the same or adjacent scan regions to achieve desired refractive index changes over larger volumes while avoiding the damage thresholds at which the materials undergo undesired changes that would degrade their optical performance. The refractive index changes written into the optical material include relatively increasing or decreasing the refractive index of the scanned regions of the optical material according to the local reaction of the optical material to the pulses delivered.

In addition to controlling the temporal and spatial relationships between the pulses of the different beams delivered to the optical materials, the characteristics of the pulses within the different beams or the different beams themselves within which the pulses are delivered can be altered or otherwise controlled. For example, the pulse energy or the pulse width of the pulses in the different beams can be relatively altered as well as the volumes through which the different beams are focused. In fact, the pulse characteristics can be relatively altered to accommodate the different size and shape volumes that can be associated with writing at extended depths in the optical material. For example, the pulses can be elongated in the direction of propagation, which allows more power to be delivered for effecting refractive index changes over an extended depth while remaining below the threshold of optical damage.

The change in refractive index that can be effected by any one dose of actinic radiation in optical materials, such as corneal tissue or hydrogels, is limited by the damage thresholds of the materials. As such, the change in refractive index is generally too small to support 2π phase changes, which are often needed to minimize phase discontinuities in Fresnel or other types of segmented optical structures written into the optical materials. However, by writing over extended depths, only one or at least fewer layers are required to be written to effect 2π phase changes. Writing the refractive index changes over extended depths makes possible faster and more accurate writing of such optical structures, as well as higher and more efficient optical performance.

### BRIEF DESCRIPTION OF THE DRAWING FIGURES

FIG. 1A is a diagram of a two-beam multiplexer that initially splits the output beam of a pulsed laser into two orthogonally polarized working beams and then recombines the two working beams for writing refractive index changes into an optical material.
FIG. 1B is a diagram of the recombined orthogonally polarized working beams of FIG. 1A showing a temporal offset between the two beams.
FIG. 2 is a diagram of a focusing system including an objective lens for converting two angularly offset working beams into two spatially offset working beams that are focused to different locations within the same transverse plane in an optical material.
FIG. 3 is a diagram of a set of parallel traces written into the optical material by scanning the focused working beams along a Y coordinate axis perpendicular to the spacing between the focal spots of the working beams in a raster pattern that indexes subsequent scans along an X coordinate axis for changing the refractive index of the optical material over the scanned region.
FIG. 4 is a similar diagram of a set of parallel traces written into the optical material by scanning the focused working beams along an X coordinate axis parallel to the spacing between the focal spots of the working beams in a raster pattern that indexes subsequent scans along a Y coordinate axis for changing the refractive index of the optical material over the scanned region.
FIG. 5 is a similar diagram of a set of parallel traces written into the optical material by scanning temporally offset working beams focused to coincident focal spots along a Y coordinate axis a raster pattern that indexes subsequent scans along an X coordinate axis for changing the refractive index of the optical material over the scanned region.
FIG. 6 is a graph showing temperature gradients in the optical material associated with the scanning of a focal spot along one of the coordinate axes.
FIG. 7 is a diagram of an alternative two-beam multiplexer with a beam shaper along the region of one of two orthogonally polarized working beams combined with a an objective lens for focusing the two working beams at different depths within an optical material.
FIG. 8 is a greatly enlarged depiction of the focal spots of FIG. 7 scanning two linear traces at different depths in the optical material.
FIG. 9 is a diagram of an alternative two-beam multiplexer with non-polarizing beamsplitters and a beam shaper in advance of an objective lens for focusing the two working beams at different depths within an optical material, wherein one of the beamsplitters for recombining the working beams includes a substantially transmissive plate with a limited reflective area to which one of the working beams is focused.
FIG. 10 is a diagram of an alternative two-beam multiplexer with non-polarizing beamsplitters and a beam shaper in advance of an objective lens for focusing the two working beams at different depths within an optical material, wherein one of the beamsplitters for recombining the working beams includes a substantially reflective plate with a central aperture through which one of the working beams is focused.
FIG. 11 is a diagram of a four-beam multiplexer combining features of the two-beam multiplexer of FIG. 1 with features of the two-beam multiplexer of FIG. 10 for focusing four working beams to two laterally offset positions in each of two vertically displaced planes.
FIG. 12 is a greatly enlarged depiction of four focal spots produced by the working beams of FIG. 11.
FIGS. 13A and 13B present graphs plotting phase change in an optical material as a function of applied optical power, demonstrating the additional optical power that can be applied to extended depth focal spots while remaining below a damage threshold of the optical material.
FIG. 14 is a diagram of a two-beam multiplexer similar to the multiplexer of FIG. 7 with the addition of two spherical aberration correction plates for elongating the focal spots produced by the two working beams.
FIG. 15 is a greatly enlarged depiction of two extended depth focal spots produced by the working beams of FIG. 14.
FIG. 16 is a graph plotting the intensities of focal spots in an axial plane for focal spots produced with zero spherical aberration, a first amount of positive spherical aberration, and a second higher amount of positive spherical aberration.
FIG. 17 is a diagram of an optomechanical scanner for translating the focal spots of any of the beam multiplexers referenced herein along three orthogonal axes for writing refractive index structures in an optical material.

### DETAILED DESCRIPTION OF THE INVENTION

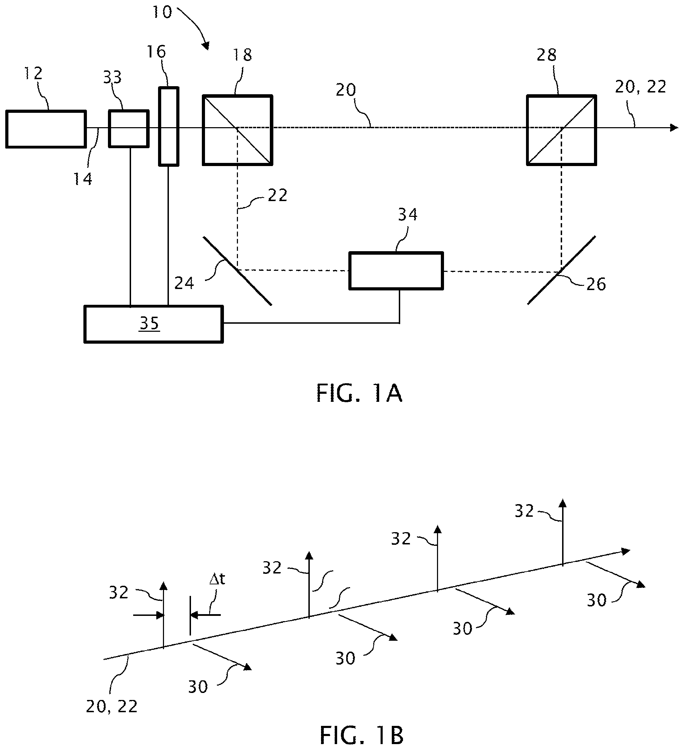

A beam multiplexer 10 as envisioned for one or more embodiments is diagramed in FIGS. 1A and 1B. A laser source 12 outputs a collimated substantially linearly polarized beam 14, whose polarization axis is rotated by a half-wave plate 16 to an orientation substantially at 45 degrees to the orthogonal polarization axes of a first polarization beamsplitter 18. The laser source 12 can be fashioned as mode-locked Ti:Sapphire laser (e.g., a Spectra-Physics Ti:Sapphire oscillator such as MaiTai-HP available from Spectra-Physics, a Newport company, in Santa Clara, California) pumped by a frequency-doubled Nd:YVO4 laser. The laser can generate, for example, a succession of pulses of up to 3 W average power, a 110 fs pulse width, and an 80 MHz repetition rate or up to 1 W average power, a 160 fs pulse width and an 80 MHz repetition rate at around 400 nm frequency-doubled wavelengths. Of course, other lasers can be used or optimized for use with writing refractive index changes into different optical materials in accordance with the marginal thresholds of the materials for undergoing localized refractive index changes without also undergoing optically induced damage such as significant light scattering or absorption that degrade their intended performance. The optical materials include ophthalmic hydrogel polymers (used in contact lenses and intraocular lenses) and cornea tissue (both excised and in vivo) as well as other ophthalmic materials that are naturally occurring or synthetically produced. It is noted that in-vivo writing methods are not a part of the invention.

Generally, for the purpose of writing refractive index structures in such optical materials, with pulsed laser sources, the succession of pulses preferably have a pulse width between 8 fs and 500 fs, a pulse energy between 0.01 nJ and 10 nJ, a repetition rate between 10 MHz and 500 MHz, and a nominal wavelength between 400 nm and 1100 nm. These parameters are also tied to the focal spot size and the scanning rate at which the focal spot is moved relative to the optical material. For writing refractive index changes over larger volumes, both the focal spot size and the scanning rate are increased as much as practically possible in coordination with the other parameters that are set to operate in an energy regime just below the damage threshold of the material. Scanning speeds up to 10 m/s are contemplated.

The first polarization beamsplitter 18 divides the collimated output beam 14 into two orthogonally polarized working beams 20 and 22. For example, the working beam 20, which transmits through the first polarization beamsplitter, has a polarization axis oriented in a horizontal plane extending out of the drawing sheet, and the working beam 22, which is reflected by the first polarization beamsplitter, has a polarization axis oriented in a vertical plane within the drawing sheet. The horizontally polarized working beam 20 propagates directly to a second beamsplitter 28. Reflectors 24 and 26 direct the vertically polarized working beam 22 to the second beamsplitter 28 in a physical orientation that remains orthogonal to the physical orientation of the working beam 20. The second polarization beam splitter 28 transmits the horizontally polarized working beam 20 and reflects the vertically polarized working beam 22 into realignment with the working beam 20.

While physically aligned as output from the second polarization beamsplitter 28, the working beams 20 and 22 remain distinguished by their relative orthogonally related polarizations as shown in FIG. 1B. Arrows depict the equally spaced timing of the femtosecond pulses 30 of the working beam 20 at a given repetition rate preferably in the MHz range, which are shown in a horizontal orientation as a further indication of the horizontal polarization orientation of the working beam 20. In a complementary fashion, arrows depict the equally spaced timing of the femtosecond pulses 32 of the working beam 22 at the given repetition rate, which are shown in a vertical orientation as a further indication of the vertical polarization orientation of the working beam 22.

Although the two working beams 20 and 22 are realigned at the output of the second polarization beamsplitter 28, the pulses 30 and 32 of the respective working beams 20 and 22 are temporally offset by Δt as a result of an optical path length difference, which can include a delay element 34 along one of the separate paths taken by the working beams 20 and 22 between the first and second polarization beamsplitters 18 and 28. Alternatively, the reflectors 24 and 26 could be collectively displaced toward or away from the first and second beamsplitters 18 and 28 with which they are aligned to change the optical path length of the working beam 22 with respect to the optical path length of the working beam 20. The temporal offset Δt can be set to vary by any amount less than the temporal spacing between the pulses as the inverse of the repetition rate. For example, at a repetition rate of 100 MHz, the pulses are temporally spaced by about 10 ns and physically spaced by 3 m. In the combined beams 20 and 22, temporal offset Δt by one half of the spacing between pulses (e.g., 5 ns at 100 MHz), the repetition rate in the combined beam is effectively doubled (e.g., 200 MHz). Other temporal offsets Δt can be used for other purposes including regulating temperature profiles in the irradiated optical materials associated with the delivery of closely adjacent pulses.

The arrows of FIG. 1B representing the pulses 30 and 32 of the respective working beams 20 and 22, while in different planes, are drawn at approximately equal lengths representative of equal pulse energies between the pulses 30 and 32. However, the pulse energies can also be unequally distributed between the pulses 30 and 32 of the respective working beams 20 and 22, such as by rotating the half-wave plate 16 to a different angular position and thereby orienting the polarization axis of the output beam 14 closer to one or the other of the orthogonal polarization axes of the first polarization beamsplitter 18 (i.e. ≠ 45°).

Thus, in addition to controlling the temporal offset Δt of the pulses 30 and 32 in the respective orthogonally polarized working beams 20 and 22, the relative pulse energies of the pulses 30 and 32 can also be controlled to regulate the delivery of energy to the optical materials intended for irradiation. Moreover, the overall pulse energies of the two beams can be controlled by a modulator 33, such as an electro-optic modulator or an acousto-optic modulator, for variably attenuating the output beam 14. The overall pulse energies and the relative pulse energies of the two working beams 20 and 22, as well as the temporal offset Δt between the pulses of the two working beams 20 and 22 can be manually controlled by settings or can be automatically controlled through a controller 35 that takes input from an operator.

The different polarization orientations of the two aligned working beams 20 and 22 also allow for subsequent angular or spatial separation of the two beams. In addition, small angular separations can be made in advance of the second polarization beamsplitter 28. For example, the reflector 26 can be tipped slightly out of a 45 degree orientation to its incident beam to relatively incline the working beam 22 with respect to the working beam 20 as output from the second beamsplitter 28.

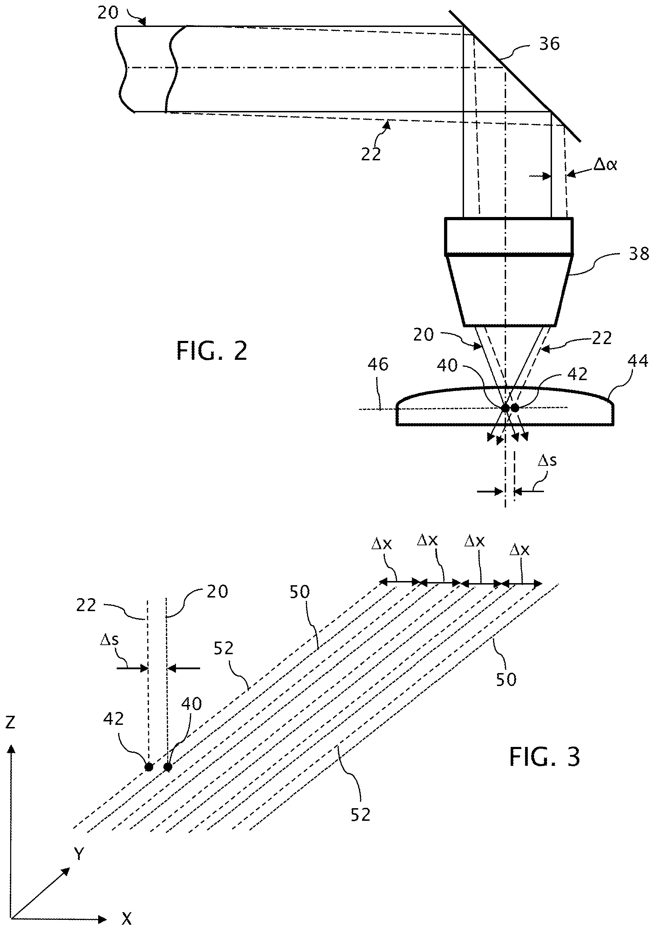

FIG. 2 shows a focusing system the two orthogonally polarized and relatively inclined working beams 20 and 22 reflected by a reflector 36 into desired alignment with an objective lens 38, which focuses the two working beams 20 and 22 to spatially offset focal spots 40 and 42 in an optical material 44. The objective lens 38, which is considered as a part of the beam multiplexer system 10, converts an angular difference Δα between the working beams 20 and 22 into a spatial offset Δs, which is measured on center between the focal spots 40 and 42. As shown, the two focal spots 40 and 42 are offset through the measure Δs in a common plane 46 oriented transverse to the optical axis 39 of the objective lens 38. In advance of the objective lens 38, the working beam 20 is aligned with the optical axis 39 and the working beam 22 is inclined to the optical axis 39 through angle Δα. However, either or both of the working beams 20 and 22 can be inclined to the optical axis 39 to produce the desired angular difference Δα. As shown, the two working beams 20 and 22 diverge in advance of the objective lens 38. However, the two working beams could also be arranged to converge at the objective lens 38 to minimize the required size of the objective lens 38 to receive both beams. Preferably, both working beams 20 and 22 have a transverse dimension (e.g., width) sufficient to substantially fill the aperture of the objective lens 38 to form highly resolved focal spots 40 and 42

The objective lens 38 can take the form of a microscope objective having a numerical aperture of preferably at least 0.28 but higher numerical apertures of 0.7 through 1.0 are often preferred if sufficient working distance is present. As respective treatment zones, the focal spots 40 and 42 occupy respective volumes of space within which the power densities of the respective beams 20 and 22 are sufficient to change the refractive index of the optical material 44 without inducing damage. Positive or negative changes in refractive index can be imparted by the respective beams 20 and 22 depending upon the reaction of the optical material to the pulses delivered by the beams.

While the focal spots 40 and 42 remain separated through the spatial offset Δs, the objective lens 38 can be moved relative to the optical material 44 in different directions to write pairs of linear traces having a line spacing ranging from zero to the spatial offset Δs. FIG. 3, for example, depicts the simultaneous writing of parallel traces 50 and 52 at a line spacing equal to the spatial offset Δs for doubling the line density that can be produced by each scan in the direction of the Y coordinate axis. The working beam 40 writes the traces 50, and the working beam 42 writes the traces 52. Each scan is indexed by an amount Δx along the X coordinate axis that is equal to twice the spatial offset Δs between the focal spots 40 and 42. The scans of the two focal spots 40 and 42 along the Y coordinate axis are repeated at different starting locations by successively shifting or otherwise indexing the focal spots 40 and 42 along the X coordinate axis by increments Δx to write the overall raster pattern shown. Of course, the dimensions depicted on the drawing figures are not to scale. For example, a typical line spacing is approximately 0.5 µm, and the angular difference Δα between the working beams 20 and 22 for such a line spacing can be in the range of 0.01 degrees. Any lesser line spacing, i.e., a line spacing less than the spatial offset Δs between the focal spots 40 and 42, can be achieved by changing the direction of the scan from a direction perpendicular to a line connecting focal spots 40 and 42 to a direction closer to parallel to the connecting line. Doubling the line density by writing two linear traces 50 and 52 at once can double the speed at which refractive structures are written into the optical material 44.

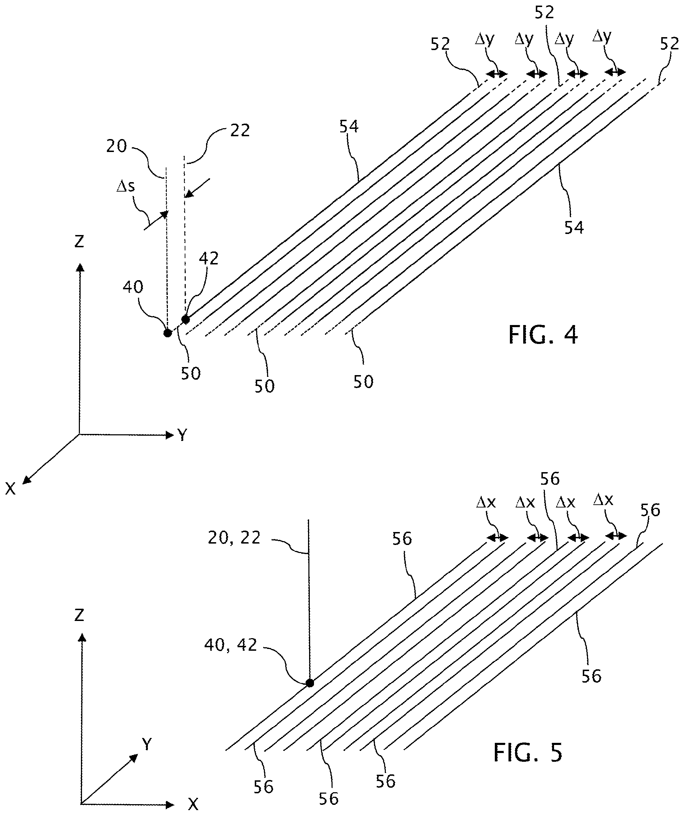

FIG. 4, for example, depicts a scan direction along the X coordinate axis parallel to a connecting line between the focal spots 40 and 42. Here, both working beams 20 and 22 trace overlapping paths for writing and rewriting the same traces 54 along each scanning path for such purposes as changing and further changing the refractive index of the optical material 44 along the traces 54. As shown in FIG. 4, each trace 54 is formed in a region where the traces 50 and 52, which are separately written by the respective focal spots 40 and 42, overlap along a common length. The traces 54 are spaced apart on center by an amount Δy corresponding to the amount each scan path is relatively indexed in the direction of the Y coordinate axis. The spatial offset Δs in the scan direction X results in a corresponding temporal offset between the respective interactions of the focal spots 40 and 42 with the optical material that depends upon the scanning speed. The corresponding temporal offset between the focal spots 40 and 42 allows heat generated by a lead focal spot, e.g., 40, to at least partially dissipate before the following focal spot, e.g. 42, generates additional heat at the same location within the optical material 44. For such purposes, the spatial offset Δs can be adjusted in relation to the scanning speed to maintain desired temperature profiles within the optical material 44.

The spatial offset Δs can be reduced to zero as shown in FIG. 5 while maintaining a desired temporal offset by Δt as shown in FIG. 1B to affect a nonsynchronous temporal spacing between pulses 30 and 32 and the overall doubling of the repetition rate. Here both working beams 20 and 22 converge to coincident focal spots 40 and 42 and contribute to forming the same set of traces 56 as traced by scan paths along the Y coordinate axis. The traces 56 are spaced on center by the index distance Δx between the scan paths. The optical path length difference between the working beams 20 and 22 can be adjusted to temporally offset the pulses 30 and 32 of the two working beams 20 and 22 through a time Δt as some fraction of the inverse of the repetition rate. When the pulses 30 and 32 are evenly spaced, the effect is temporally equivalent to doubling the repetition rate. However, when unevenly spaced, successive pairs of the pulses 30 and 32 can be delivered in groups within which the pulses 30 and 32 of each group are delivered in more rapid succession. If the pulses 30 and 32 within each group are spaced apart in the vicinity of the pulse width, the affect can be similar to enlarging the pulse width. However, at slightly larger temporal offsets Δt, the successive pulses 30 and 32 of each group can be used to further control energy transfer profiles within local volumes of the optical material 44. In addition, the relative amounts of pulse energy in the successive pulses 30 and 32 can be controlled, such as by changing the orientation of the half-wave plate 16, to further control the energy transfer profiles within the local volumes. For example, the energy transfer profiles can be set to both raise and maintain the accumulation of energy within the local volumes above the minimum threshold required for changing the refractive index of the optical material 44 and below the maximum threshold at which the intended optical performance of the optical material 44 is adversely affected. In this regard, the second of the successive pulses 30 and 32 within each group can be provided with less pulse energy than the first of the successive pulses 30 and 32 within each group to optimize the desired nonlinear absorption characteristics within the localized volumes of the optical material 44 without overheating or otherwise damaging the optical material 44.

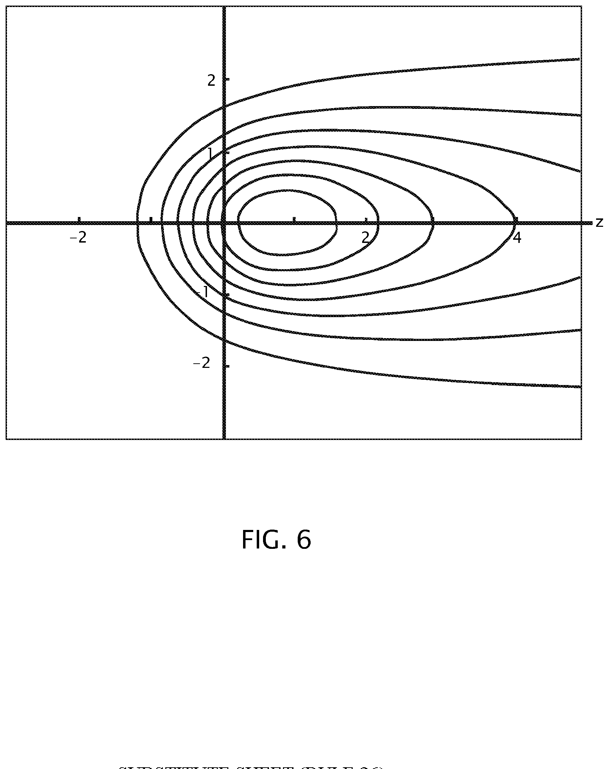

FIG. 6 depicts a temperature profile within an optical material as a focal spot is scanned across the material from right to left. The temperature increases are more abrupt and concentrated at the leading edge of the focal spot and the resulting temperature gradients tend to persist and spread following the passage of the focal spot. Thus, the energy characteristics, e.g., pulse energy, pulse width, and repetition rate, as well as the spatial offset Δs and scan speed of a following focal spot must be arranged to account for any residual heat profiles in the optical material as a different base level to which the energy of the following focal spot is added. Similar considerations also apply when the spatial offset Δs includes a component that is traverse to the scanning direction.

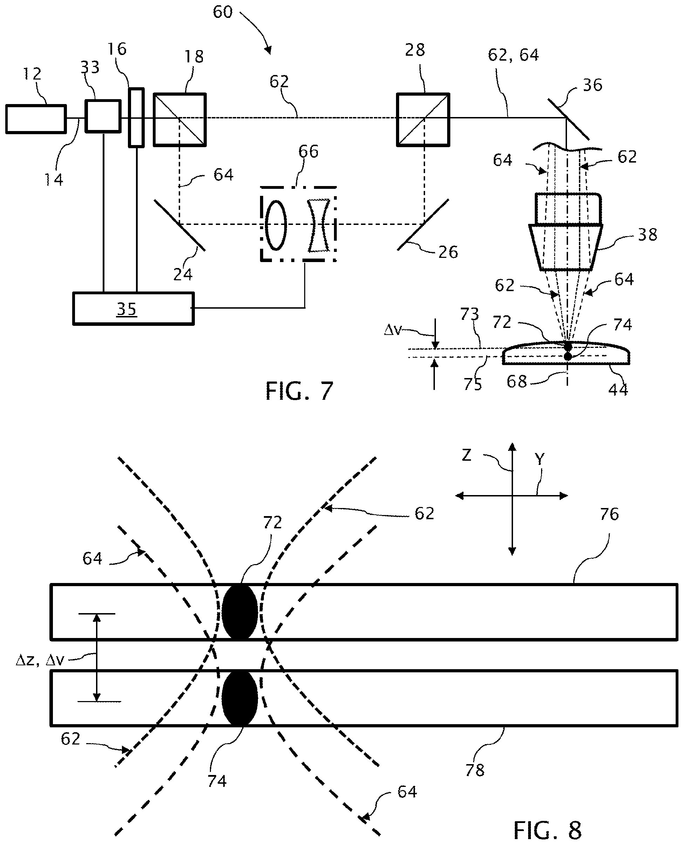

An alternative beam multiplexer 60 is shown in FIG. 7 incorporating a focusing system for writing at multiple depths within an optical material. Various elements in common with the beam multiplexer 10 share the same reference numerals. For example, similar to the beam multiplexer 10, the laser source 12 outputs a collimated substantially linearly polarized beam 14, whose polarization axis is rotated by a half-wave plate 16 to a desired orientation with respect to the orthogonal polarization axes of a first polarization beamsplitter 18. The first polarization beamsplitter 18 divides the collimated output beam 14 into two orthogonally polarized working beams 62 and 64. Departures from a 45 degree orientation of the half-wave plate 16 can be used to adjust the relative pulse energies of the two working beams 62 and 64. The modulator 33 can be used to modulate the overall intensity of the output beam 14, and together with the adjustable half-wave plate 16 under the optional control of the controller 35, can adjust both the absolute and relative pulse energies of the two working beams 62 and 64.

The working beam 62, which has a first orthogonal polarization propagates directly to a second beamsplitter 28. Reflectors 24 and 26 direct the working beam 64, which has a second orthogonal polarization to the second beamsplitter 28. However, along the optical region between the reflectors 24 and 26, a beam shaping optic 66, which can take the form of a telescope, reshapes the working beam 64 from a substantially collimated form to a converging or diverting form. The amount of the divergence or convergence can be set manually or placed under the control of the controller 35.

A focusing system 66 similar to the focusing system of FIG. 3 is also shown at the output of the second beamsplitter 28 and includes the reflector 36 and the objective lens 38. The reflector 35 is representative of the optical elements required to convey the collimated working beam 62 and the converging or diverging working beam 64 to the objective lens 38. For purposes of providing a clearer illustration, a break is provided between the reflector 36 and the objective lens 38 at which the working beam 62 is depicted as a collimated beam and the working beam 64 is depicted as a diverging beam approaching the objective lens 38. While also for purposes of illustration, the transverse dimension of the collimated working beam 62 is shown smaller than the traverse dimension of the diverging working beam 64, both working beams 62 and 64 are preferably sized to substantially fill the aperture of the objective lens 38.

The collimated working beam 62 is brought to focus at a focal spot 72 at the focal length of the objective lens 38. The diverging working beam 64 is brought to focus at a focal spot 74 that is located farther from the objective lens 38, which applies the same power of convergence too both working beams 62 and 64. The two focal spots 72 and 74 are located in transverse planes 73 and 75 that are spaced apart along the optical axis 68 of the objective lens 38 through a spatial offset Δv. While for purposes of illustration, the transverse dimension of the collimated working beam 62 is shown smaller than the traverse dimension of the diverging working beam 64, both working beams 62 and 64 are preferably sized to substantially fill the aperture of the objective lens 38. For example, the beam shaper 66 can be used to produce a beam that converges through a focus in advance of the objective lens 38 so that the diverging beam approaches the objective lens 38 at a desired size.

FIG. 8 is a greatly enlarged schematic depiction of the working beams 62 and 64 focused though focal spots 72 and 74 for writing linear traces 76 and 78 at different depths along the Z coordinate axis within the optical material 44. A centerline spacing Δz between the traces 76 and 78 corresponds to the spatial offset Δv at which the working beams 62 and 64 are brought to focus through their respective focal spots 72 and 74. The traces 76 and 78 are shown scanned along the Y coordinate axis, and an array of linear trace pairs 76 and 78 can be formed by repeating the Y-axis scan at an index spacing Δx along the orthogonal X axis as shown for example in FIG. 5.

The spacing Δz corresponding to the spatial offset Δv between the focal spots 72 and 74 can be set for writing contiguous layers of refractive index change or for writing different layers whose refractive index change is partially or completely independent of one another. The spatial offset Δv in depth can be combined with other temporal and spatial offsets. For example, the focal spots 72 and 74 can be formed by pulses in their respective working beams 62 and 64 that are temporally offset by the time Δt so that the pulses (e.g., 30 and 32) of both working beams do not traverses the upper layer (e.g., trace 76) at exactly the same time as described in connection with FIG. 1B and 5. In addition, the focal spots 72 and 74 can also be spatially offset in the X-Y plane for further spatially separating the focal spots 72 and 74 in accordance with the spatial offset (e.g., Δs) and the relative direction at which the scan is taken relative to the X and Y coordinate axes as described in connection with FIG. 3 and for further temporally separating the focal spots in accordance with the spatial offset (e.g., Δs) and the scanning speed as described in connection with FIG. 4. The relative pulse energies of the working beams 62 and 64 can also be adjusted for such purposes as compensating for different purposes as compensating for different required writing parameters associated with writing at different depths. For instance, different spherical aberration plates would require different powers.

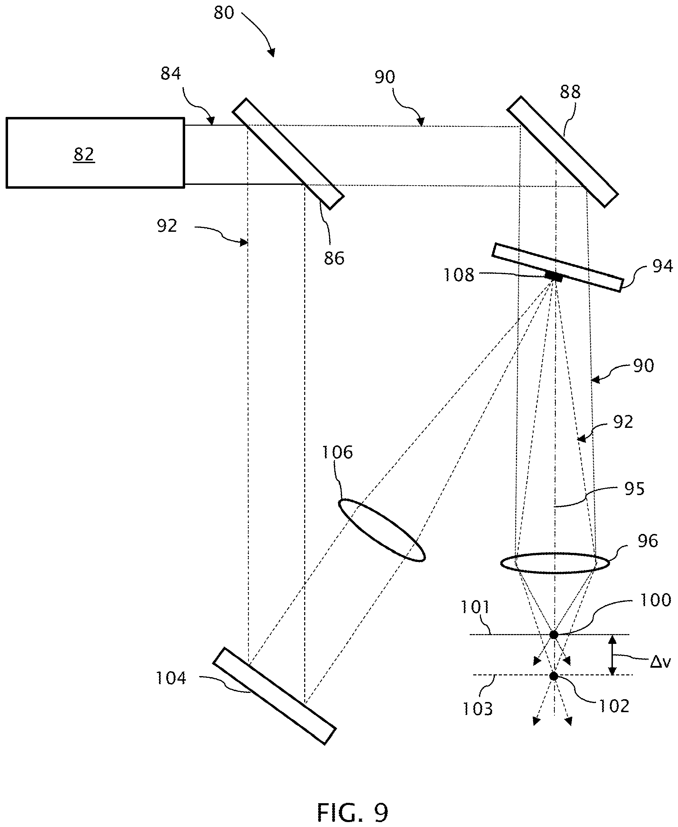

An alternative two-beam multiplexer system 80 is depicted in FIG. 9 for simultaneously writing at different depths in an optical material. A laser source 82, preferably in the form of a pulsed laser of the type described above, emits a collimated beam 84 that is divided by a beamsplitter 86 into a first collimated working beam 90 that transmits through the beamsplitter 86 and a second collimated working beam 92 that reflects from the beamsplitter 86. The beamsplitter 86 can be arranged in a number of ways, including as a polarization beamsplitter as described earlier or as a partial reflector with metallic, e.g., "half-silvered" surface. The ratio of transmission to reflection can be controlled in number of known ways to regulate the respective pulse energies of the two working beams 90 and 92.

The collimated working beam 90 is reflected by a reflector 88 through a substantially transmissive plate 94 to an objective lens 96, which focuses the working beam 90 along the optical axis 95 of the objective lens 96 to a focal spot 100 in a transverse plane 101 at approximately one focal length from the objective lens 96. The collimated working beam 92 is reflected by a reflector 104 through a converging lens 106 that focuses the working beam 92 onto a central reflector 108 mounted on the substantially transmissive plate 94 at approximately one focal length from the lens 106. After reflecting from the central reflector 108, the working beam 92 further propagates as a diverging beam to the objective lens 96. Together, the substantially transmissive plate 94 and the central reflector 108 function as another beamsplitter for overlapping the two working beams 90 and 92 en route to the objective lens 96. However, the objective lens 96 focuses the diverging working beam 92 along the optical axis 95 to a focal spot 102 that is located in a transverse plane 103 at a farther distance from the objective lens 96 than the focal spot 100 by a spatial offset Δv.

The amount of spatial offset Δv can be adjusted in a number of ways, including by changing the focal length of the converging lens 106 and correspondingly adjusting the positions of the converting lens 106 and the substantially transparent plate 94 together with the central reflector 108. The central reflector 198, which is preferably elliptically shaped because of the angular orientation of the substantially transmissive plate 944 to the converging working beam 92, blocks a small percentage of the light of the working beam 90 within its paraxial zone. The beamsplitter 86 can be arranged to compensate for this relative loss or other relative losses elsewhere in the system and to set the relative pulse energies of the two working beams 90 and 92 as desired to achieve the desired energy profiles in the optical material. To compensate for any unwanted aberrations affecting the size or shape of the focal spot 102, the converging lens 106 can be formed as an aspheric optic or one or more other compensators can be located along the optical region to the objective lens 96.

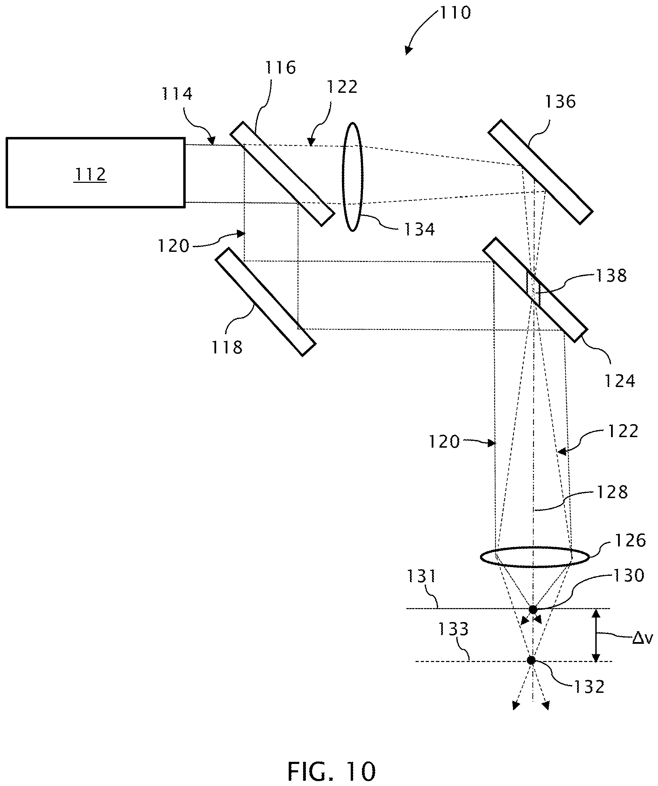

Another two-beam multiplexer system 110 is shown in FIG. 10 for simultaneously writing at different depths in an optical material. A similar pulsed laser source 112 emits a collimated beam 114 that is divided by a beamsplitter 116 into a first collimated working beam 120 that reflects from the beamsplitter 116 and a second collimated working beam 122 that transmits through the beamsplitter 116. The beamsplitter 116 can be arranged in a number of ways as described above. The ratio of transmission to reflection can be controlled in number of known ways as described above to regulate the respective pulse energies of the two working beams 120 and 122.

The collimated working beam 120 is reflected by a reflector 118 to a substantially reflective plate 124 that reflects the working beam 120 to an objective lens 126, which focuses the working beam 120 along an optical axis 128 to a focal spot 130 in a transverse plane 131 at approximately one focal length from the objective lens 126. The collimated working beam 122 is focused by a converging lens 134 into a converging beam that is reflected from a reflector 136 to a focus within a central aperture 138 of the substantially reflective plate 124 at a distance of one focal length from the converging lens 134. The focused converging beam further propagates from the focus as a diverging beam to the objective lens 126. Thus, the substantially reflective plate 124 together within is central aperture functions as another beamsplitter for recombining the two working beams 120 and 122 en route to the objective lens 126. The working beam 122 is focused by the objective lens 126 along the optical axis 128 to a focal spot 132, which is located at a farther distance from the objective lens 126 than the focal spot 130 by a spatial offset Δv. As explained above, the amount of spatial offset Δv can be adjusted in a number of ways, including by changing the focal length of the converging lens 134 and correspondingly adjusting the positions of the converting lens 134 and the substantially reflective plate 124 together with its central aperture 138. The central aperture 138, which is preferably elliptically shaped because of the angular orientation of the substantially reflective plate 124 to the converging working beam 122, excludes a small percentage of the light of the working beam 120 from reflecting toward the objective lens 126. The beamsplitter 116 can be arranged to compensate for this relative loss or for other purposes to set the relative pulse energies of the two working beams 120 and 122 as desired to achieve the desired energy profiles in the optical material. To compensate for any unwanted aberrations affecting the size or shape of the focal spot 132, the converging lens 134 can be formed as an aspheric optic or one or more other compensators can be located along the optical region to the objective lens 126.

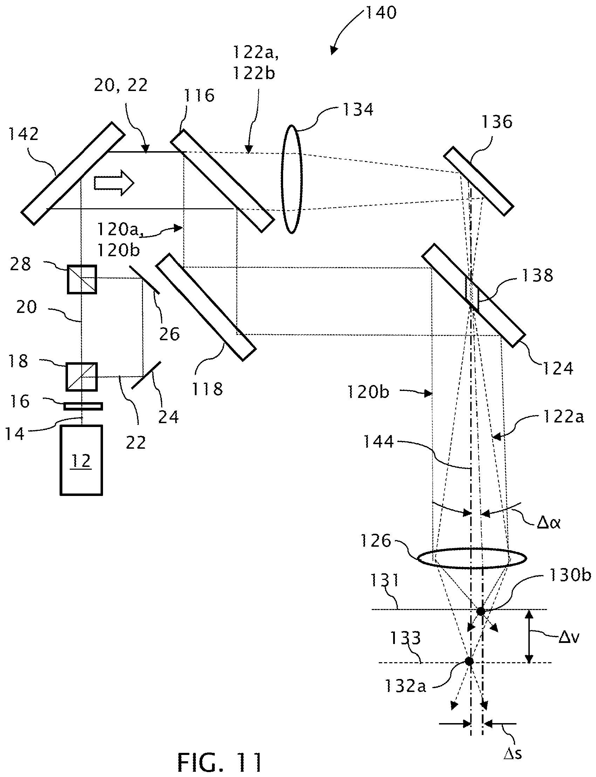

A four-beam multiplexer system 140 is shown in FIG. 11 combining the multiplexer system 10 of FIG. 1A with the multiplexer system 110 of FIG. 10 for simultaneously writing at both different positions and different depths in an optical material. Elements substantially in common with the multiplexer systems 10 and 110 are designated by the reference numerals of the earlier described systems.

The pulsed laser source 12 outputs a collimated substantially linearly polarized beam 14, whose polarization axis is rotated by a half-wave plate 16 to a desired orientation with respect to the orthogonal polarization axes of a first polarization beamsplitter 18. The first polarization beamsplitter 18 divides the collimated output beam 14 into two orthogonally polarized working beams 20 and 22. Departures from a 45 degree orientation of the half-wave plate 16 can be used to adjust the relative pulse energies of the two working beams 20 and 22. The working beam 20, which has a first orthogonal polarization propagates directly to a second beamsplitter 28. Reflectors 24 and 26 direct the working beam 22, which has a second orthogonal polarization to the second beamsplitter 28. However, One of the encounters with the reflectors 24 or 26, the beamsplitter 28, or a subsequent polarization sensitive optic relatively angularly inclines the working beam 22 with respect to the working beam 20. Of course, either or both working beams can be inclined to achieve the desired angular relationship.

The relatively inclined working beams 20 and 22 are directed by a reflector 142 to a third beamsplitter 116, which divides the working beams 20 and 22 into first and second collimated working beams 120a and 120b that reflect from the beamsplitter 116 and third and fourth collimated working beams 122a and 122b that transmit through the beamsplitter 116. The respective pulse energies in each of the four working beams can be controlled in a number of ways including adjusting the angular orientation of the half-wave plate 16 and the relative reflectivity of the beamsplitter 116. The modulator 33 can also be added as well as the controller 35 for controlling various parameters of the working beams 120a, 120b, 122a, and 122b based on operator input.

The collimated working beams 120a and 120b are reflected by the reflector 118 to the substantially reflective plate 124 that reflects the working beams 120a and 120b to the objective lens 126. For clarity of illustration, only the working beam 120b is depicted en route to the objective lens 126. The working beam 120b is shown inclined through the angular offset Δα, which is referenced against an optical axis 144 of the objective lens 126. The objective lens 126 focuses the working beam 120b in the transverse plane 131 to a focal spot 130b that is located at approximately one focal length from the lens 126 but is spatially offset from the optical axis 144 by the amount Δs. The collimated working beams 122a and 122b are focused by a converging lens 134 into converging beams that are reflected from the reflector 136 to respective focuses within the central aperture 138 of the substantially reflective plate 124 at a distance of approximately one focal length from the converging lens 134. The focused converging beams 122a and 122b further propagate from their focus positions as diverging beams to the objective lens 126. Again, for clarity of illustration, only the diverging working beam 122a is depicted en route to the objective lens 126. The working beam 122a, which is oriented along the optical axes 144 is brought to a focus by the objective lens 126 within the transverse plane 133 at a focal spot 132a, which is located along the optical axis 144 at a distance beyond the focal spot 130b by the spatial offset Δv. Although not shown in FIG. 11, the working beam 120a is preferably focused by the objective lens 126 to a focal spot 130a that is located along the optical axis 144 at a distance from the objective lens 126 within the transverse plane 131 similar to the focal spot 130b. Similarly, the working beam 122b is preferably focused by the objective lens 126 to a focal spot 132b within the transverse plane 133 at a distance from the objective lens 126 similar to the focal spot 132a but at spatial offset Δs to the optical axis 144.

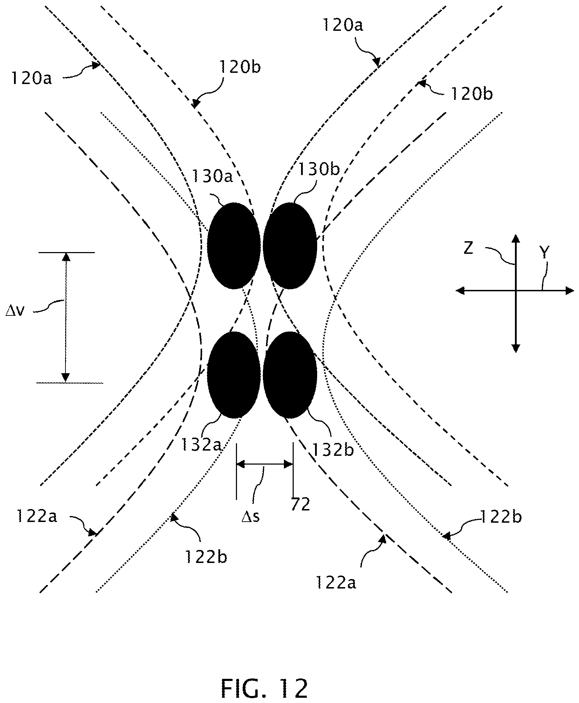

In the same plane as FIG. 11, but in a greatly enlarged view, FIG. 12 shows the four working beams 120a, 120b, 122a and 122b through positions of focus at which the focal spots 130a, 130b, 132a, and 132b are generated. The focal spots 130a and 132a are separated from the focal spots 130b and 132b along the Y coordinate axis by the spatial offset Δs. The focal spots 130a and 130b are separated from the focal spots 132a and 132b along the Z coordinate axis by the spatial offset Δv. In this configuration, two traces at each of two depths can be scanned into the optical material. Alternatively, a single trace can be scanned at each of two depths with each trace being scanned by two beams. Depending on the selected offsets Δs and Δv and the scanning direction, refractive index changes can be written in any combination of separate traces or contiguous regions within the optical material. In addition, the spatial offsets Δs and Δv, and scanning direction (and scanning speed) can be combined with temporal offsets as well as pulse energy distributions among the working beams 120a, 120b, 122a and 122b to achieve and maintain energy profiles within the optical material at the marginal thresholds of the optical materials for undergoing localized refractive index changes without also undergoing breakdowns such as significant light scattering or absorption that degrade their intended performance.

Working beams can also be spatially offset by multifocal lens designs. Multiple focal lengths can be achieved by compound refractive or diffractive optics as well as by combinations of refraction and diffraction within the same optics. The amount of energy delivered to focal spots at different focal lengths can be controlled as well as the number of different focal spots at different focal lengths.

In addition to regulating the temporal and spatial offsets between different trains of pulses, the shape of pulses can also be controlled to improve writing performance in optical materials. For example, pulses can be elongated in their direction of propagation by various focusing techniques including the introduction of spherical aberration. Significantly, such pulse elongation can extend the volume of material within which individual focal spots can impart refractive index changes. More optical power can be delivered within the extended volumes enabling a change in refractive index through a greater depth without exceeding the damage threshold of optical materials.

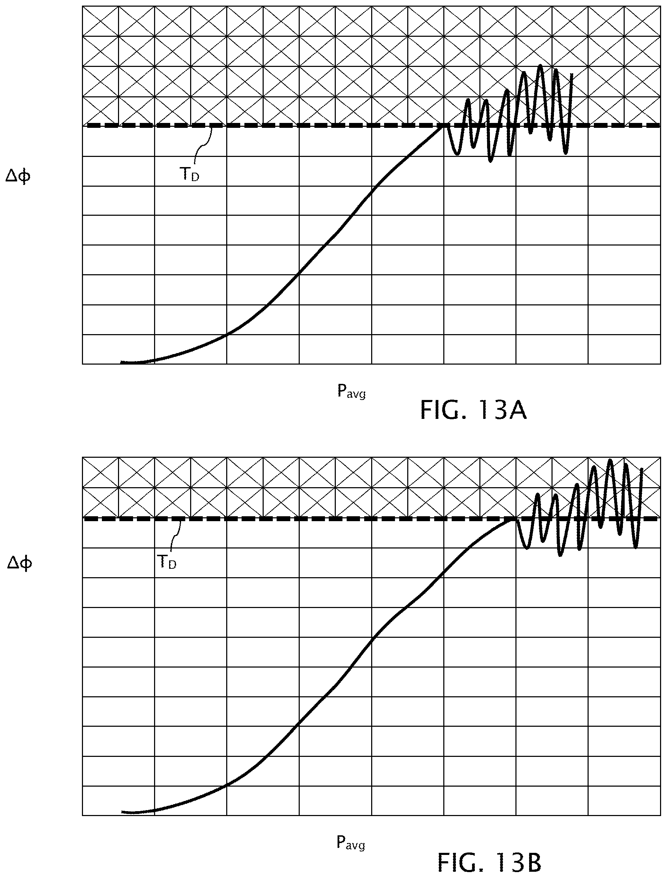

The graphs of FIGS. 13A and 13B compare exemplary plots of a change in phase Δϕ over a range of increasing average optical power P_{avg} for differently shaped pulses. The change in phase Δϕ is the desired temporal effect on the propagation of light through the optical material associated with the change in refractive index over an extended depth within the optical material 44. The change in phase Δϕ for a given wavelength is based on a product of the change in refractive index and the depth over which the index change is written (e.g., Δϕ = (2π Δn d) / λ; where Δn is the change in refractive index, d is the distance through which light travels through the changed refractive index material, and λ is the wavelength of the light). Together, the product of the change in refractive index Δn and the physical distance d through which light travels through the changed refractive index material equals the optical path difference (OPD), which is the basis for the change in phase Δϕ experienced by light propagating through the optical material. Calibrated phase changes Δϕ can contribute to desired reshaping of wavefronts propagating through the optical material for contributing optical power or controlling optical aberrations. The average power P_{avg} can be calculated from the product of the pulse energy and the repetition rate (e.g., P_{avg} = Eₚ Rᵣₐₜₑ; where Eₚ is the energy per pulse and the Rᵣₐₜₑ is the repetition rate).

FIG. 13A shows a progressive increase in the change in phase Δϕ with the application of increasing average power P_{avg} delivered to an optical material within a conventional focal spot produced without significant spherical aberration or other focal spot elongation. At a certain average power P_{avg}, a damage threshold T_{D} is reached at which the effect on phase change Δϕ becomes erratic. FIG. 13B shows that before the damage threshold T_{D} is reached within a focal spot reshaped by spherical aberration or other focal spot elongation, additional average power P_{avg} can be delivered to the optical material to effect a further increase in the total change in phase Δϕ. That is, the elongated focal spot can receive more average power P_{avg} and effect a greater phase change Δϕ than a focal spot that is not similarly elongated.

While the change in refractive index that can be effected by any one dose of actinic radiation in optical materials is limited by the damage thresholds of the materials, elongated focal spots can increase the change in phase supported by the change in refractive index by extending the change in refractive index through a greater depth. With conventionally formed focal spots, the change in refractive index is often too small to support 2π phase changes, which are often needed to minimize phase discontinuities in Fresnel or other types of segmented optical structures written into the optical materials. However, by elongating the focal spots over extended depths, only one or at least fewer layers need to be written to effect 2π phase changes. Thus, writing the refractive index changes over extended depths enables the faster and more accurate writing of such optical structures and can result in higher and more efficient optical performance.

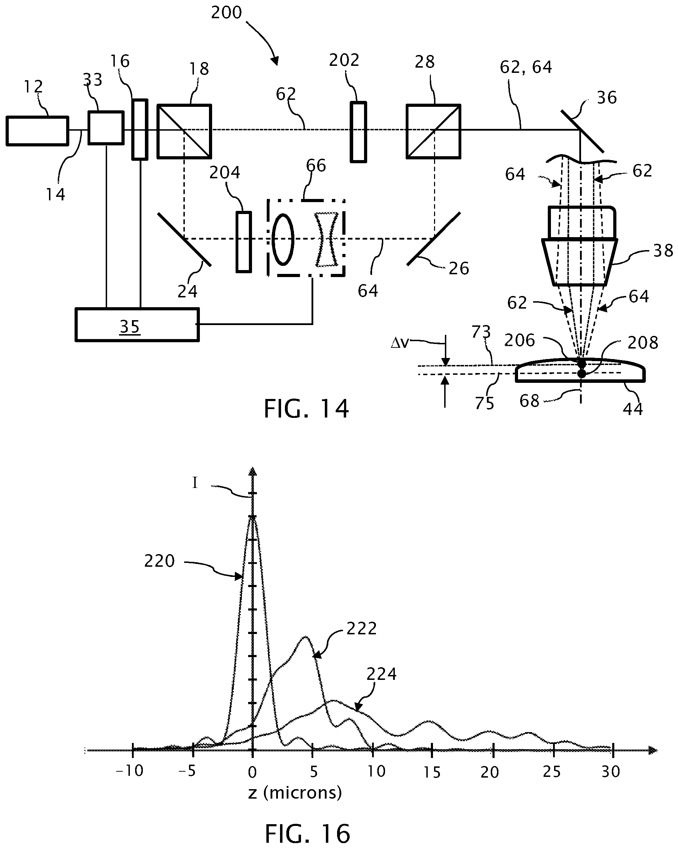

A beam multiplexer arrangement 200 shown in FIG. 14 shares a number of components in common with the multiplexer arrangement 60 shown in FIG. 7 as indicated by their reference numerals in common. In addition to the components in common, the multiplexer arrangement 200 includes two beam shapers in the form of spherical aberration compensation plates 202 and 204 along the respective pathways of the two orthogonally polarized working beams 62 and 64 in locations at which the two beams 62 and 64 propagate in a substantially collimated form. However, instead of adding respective measures of spherical aberration to compensate for other unwanted sources of spherical aberration affecting the working beams 62 and 64, the spherical aberration compensation plates 202 and 204 introduce spherical aberration that remains wholly or at least partially uncorrected for producing elongated focal spots 206 and 208 in the optical material 44 in the direction of beam propagation. Both spherical aberration compensation plates 202 and 204 introduce positive spherical aberration or negative spherical aberration or one of the plates 202 or 204 can introduce positive spherical aberration and the other of the plates 202 or 204 can introduce negative spherical aberration. In addition, it is possible to introduce a combination of positive and negative spherical aberration. Alternatively, only one or the other spherical aberration compensation plates 202 or 204 can be used so that just one or the other of the focal spots 206 or 208 is elongated by spherical aberration. Such spherical aberration compensation plates are available from Edmund Optics Inc. of Barrington, New Jersey under the tradename TECHSPEC^{®} Spherical Aberration Plate.

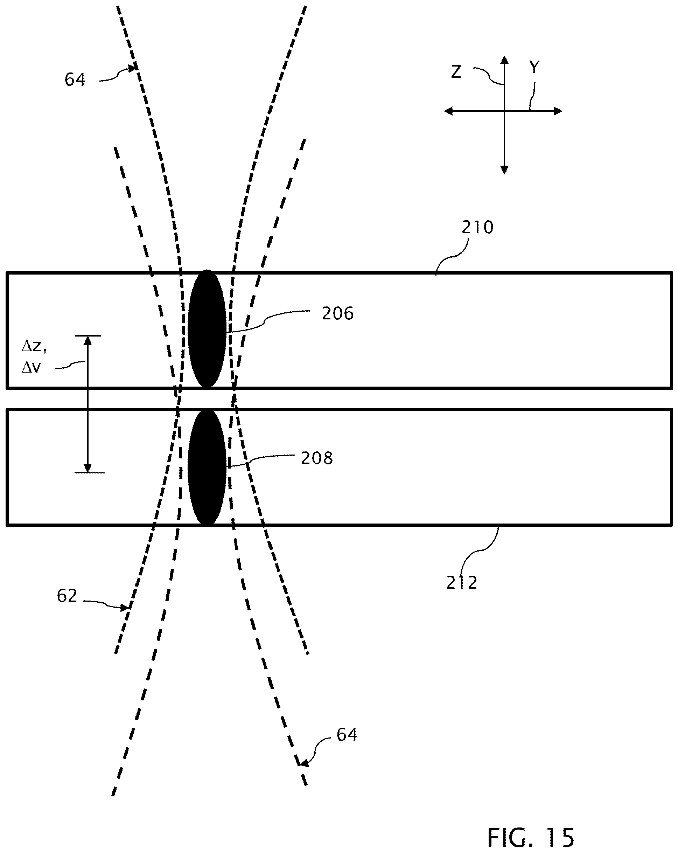

In the greatly enlarged axial view FIG. 15, the elongated focal spots 206 and 208 write linear traces 210 and 212 characterized by local changes in refractive index at different depths along the Z coordinate axis within the optical material 44. The centerline spacing Δz between the traces 210 and 212 corresponds substantially to the spatial offset Δv at which the working beams 62 and 64 are brought to focus through their respective focal spots 206 and 208. The traces 210 and 212 are shown scanned along the Y coordinate axis, and an array of linear trace pairs 210 and 212 can be formed by repeating the Y-axis scan at an index spacing Δx along the orthogonal X axis as shown for example in FIG. 5.

Also similar to other embodiments, the spacing Δz corresponding to the spatial offset Δv between the focal spots 206 and 208 can be set for writing contiguous layers of refractive index change or for writing different layers whose refractive index change is partially or completely independent of one another. The spatial offset Δv in depth can be combined with other temporal and spatial offsets. For example, the focal spots 206 and 208 can be formed by pulses in their respective working beams 62 and 64 that are temporally offset by the time Δt so that their respective pulses do not traverse the upper layer (e.g., trace 210) at exactly the same time. In addition, the focal spots 206 and 208 can be spatially offset in the X-Y plane for further spatially separating the focal spots 206 and 208 in accordance with the spatial offset (e.g., Δs) and the relative direction at which the scan is taken relative to the X and Y coordinate axes as described in connection with FIG. 3. The focal spots 206 and 208 can be further temporally and spatially offset in accordance with the spatial offset (e.g., Δs) and the scanning speed as described in connection with FIG. 4. The relative pulse energies of the working beams 62 and 64 can also be adjusted for such purposes as compensating for different loses associated with writing at different depths.

However, in contrast to the preceding embodiments, the focal spots 206 and 208 are elongated along the Z axis as a result of the spherical aberration added to the working beams 62 and 64. The output power of the laser 12 transmitted by the working beams 62 and 64 is increased as a function of the added spherical aberration so that the power received by the focal spots 206 and 208 remains above the nonlinear absorption threshold required to induce a desired change in the refractive index in the optical material 44 throughout at least a portion of the extended depths of focus while remaining below the breakdown threshold of the optical material 44. Preferably, the traces 210 and 212 are each written at a thickness T (corresponding to the elongated depth dimension of the focal spots 206 and 208) that is sufficient within the traces 210 and 212 individually or collectively to support a 2π phase change for a nominal wavelength intended for propagation through the optical material 44. For example, at least one of the traces has a thickness T capable of supporting the 2π phase change. Alternatively, the traces 210 and 212 as written in pairs of traces can have a combined thickness (e.g. 2T) capable of supporting the 2π phase change. Either way, the collective focusing the working beams 62 and 64 result in the 2π phase change as the objective lens 38 is relatively moved with respect to the optical material 44. For many vision systems, the nominal wavelength is expected to be around 550 nm, and a 0.02 index change would require a trace having a thickness of approximately 27.5 microns.

The shapes of focal spots, such as the focal spots 206 and 208, can be elongated in a plane of propagation, i.e., in an axial plane by the introduction of positive or negative aberration. Positive spherical aberration tends to elongate the focal spots in a direction opposing the direction of propagation and negative spherical aberration tends to elongate the focal spots in the direction of propagation. That is, the added spherical aberration has the overall effect of shifting and reducing optical intensity at a peak focus while relatively increasing optical intensity in positions axially offset from the peak focus. Positive and negative spherical aberrations tend to shift the peak focus in opposite directions, which can be used to further control the depths and axial spacing at which the respective traces 210 and 201 are written.

In FIG. 16, exemplary axial intensity distributions along the Z axis of focal spots are plotted for three different amounts of spherical aberration. The data is representative of an objective lens with a numerical aperture of 0.7 for conveying a 405 nm wavelength beam having a 6 mm beam diameter in a Gaussian form. Plot 220 represents an axial intensity distribution of a focal spot with no spherical aberration and appears as a conventional Gaussian focus centered at z = zero within the nominal focal plane. Plot 222, shown in dashed line, represents an axial intensity distribution of a focal spot exhibiting a first magnitude of negative spherical aberration (e.g., 2 microns of spherical aberration). Plot 224, shown in dotted line, represents an axial intensity distribution of a focal spot exhibiting a second higher magnitude of negative spherical aberration (e.g., 6 microns of spherical aberration). In general, the peaks of the intensity distributions tend to diminish and shift in a direction away from the light source as the magnitude of the negative spherical aberration increases. In addition, the intensity tends to distribute more widely through the depth of focus as spherical aberration becomes more pronounced.

An increase in optical power delivered to the extended depth focal spots not only restores the desired optical intensity near a peak focus for writing refractive index changes in the optical material 44 without inducing optical damage, the increase in optical power also elevates the intensities to either side of the peak focus above the nonlinear threshold for writing the desired refractive index changes throughout a greater depth of the optical material. By increasing the overall amount of optical power delivered to the elongated focal spots, the axial intensity distribution associated with a spherically aberrated focal spot can be elevated above the nonlinear absorption threshold T_{A} over an extended depth while remaining below the damage threshold T_{D}.

While the spherical aberration compensation plates 202 and 204 provide a ready way of introducing spherical aberration in collimated beams, a variety of other ways are known to introduce spherical aberration, including within converging or diverging beams. For example, simple plane parallel plates of varying optical thickness can be used in non-collimated beams to introduce varying amounts of spherical aberration by refracting marginal rays more than paraxial rays. Certain lenses are also available with corrector rings that can be can be used to adjust spherical aberration within the lenses.

In addition, while spherical aberration is an optical parameter that is relatively easy to control by beam shaping optics, a variety of other ways can be used to produce extended depth focal spots. For example, non-diffraction-limited beams, which generally focus to a larger spot size can also be arranged to expand along the depth of focus, i.e., in a paraxial approximation, as an expansion of the Rayleigh range from the beam waist to the point where the area of the beam cross section is doubled. For example, beam shapers of various types can be used including apodizers and diffractive optics.

The extended depth focal spots that are elongated in the direction of propagation are particularly useful for writing in ophthalmic materials with femtosecond lasers, particularly at high numerical apertures (e.g., above .28) and at high repetition rates (e.g., above 10 MHz). The extended depth focal spots allow for the more efficient use of optical power in ophthalmic materials where doses of laser radiation are limited for safety purposes. By writing over larger volumes within high numerical apertures at high speeds, the doses of laser power delivered to patients can be reduced.

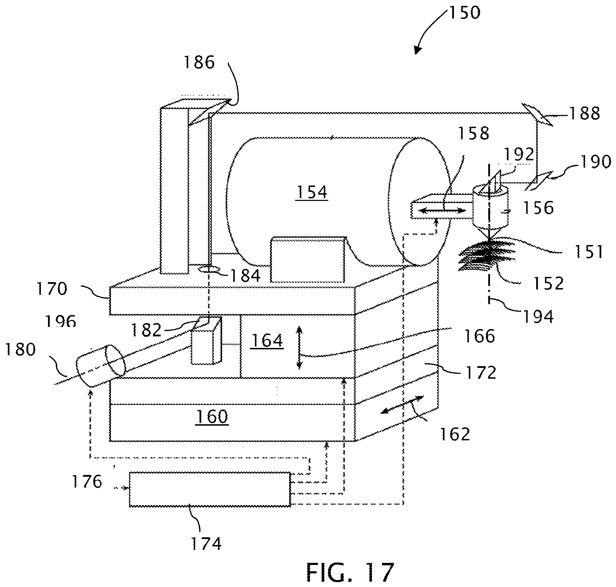

FIG. 17 is a schematic diagram of an optomechanical scanner 150 for writing refractive structures 152 within an optical material with stacked stages to provide for relatively moving a focusing system along three coordinate axes with respect to the optical material. The optomechanical scanner 150 includes a reciprocal shaker (e.g. a rapidly shaking impeller) as a fast axis scanner 154 that provides for rapidly translating an optics assembly 156 along a first scanning motion axis 158. The optics assembly 156 includes an objective lens similar to the objective lenses of the preceding embodiments for focusing working beams into an optical material. A high speed depth control stage and a spherical aberration correction stage can also be incorporated into the optics assembly 156. The high speed depth control can correct for angular motion errors to ensure and the spherical aberration stage can be used to correct for spherical aberrations to improve focal spot quality. One or more focal spots 151 of the working beams are directed along scan paths in the optical material as imparted by the optomechanical scanner 150.

The optomechanical scanner 150 also includes a motion stage 160 for translating both the optics assembly 156 and the fast axis scanner 154 along a second scanning motion axis 162, which is oriented orthogonal to the first scanning motion axis 158. The motion stage 160 can be arranged to provide continuous or stepped motions in synchronism with the motion imparted by the fast axis scanner 154. A precision height stage 164 is interposed between the motion stage 160 and the a fast axis scanner 154 to raise and lower the fast axis scanner along a third scanning motion axis 166 for such purposes as controlling the depth at which the focal spots 151 are written into the optical material.

The optomechanical scanner 150 is particularly arranged for moving the optics assembly 156 with respect to the optical material, which can be particularly useful for in-vivo applications where the optical material cannot be as easily moved. However, for other applications or considerations, the motion axes can be distributed between the optics assembly 156 and the optical material in any combination, and one or more additional motion axes, including rotational axes, can be added as required.

The fast axis scanner 154 can be a commercial vibration exciter to provide high speed reciprocal motion. One example of such a commercial vibration exciter is a Brüel and Kjaer Measurement Exciter Type 4810 sold by Brüel & Kjaer Sound & Vibration Measurement A/S of Nærum, Denmark. The motion stages 160 and 164 can be a high-precision linear stages, such as model GTS70 for lateral motion and model GTS20V for vertical motion from the Newport GTS Series, sold by Newport Corporation of Irvine, California and adapted via appropriate interface plates 170 and 172 for stacking the motion axes.

Motions along the various axes 158, 162 and 164 can be controlled by an arrangement of controllers and amplifiers 174 that translate inputs 176 in the form of desired writing patterns into motions along the various axes 158, 162, and 164. For example, the fast axis scanner 154 can be controlled by an arbitrary waveform generator. Such waveform generators are sold by Agilent Technologies, Inc. of Santa Clara, California. The waveform for the motions along the first scanning motion axis 158 are arranged, for example, to result in the desired refractive index pattern along the first scanning motion axis 158. Instead of sending an arbitrary waveform to the fast axis scanner 154, a specially tuned sine wave can be sent to maximize performance. For example, the drive frequency can be tuned to a resonance frequency of the fast axis scanner 154 to enable high speed motion while inducing minimal disturbances into the supporting structures including the underlying motion stages 160 and 166.

The working beams 180 are aligned and steered along each axis of motion to ensure proper alignment of the working beams 180 with the optics assembly 156. For example a reflector 182 mounted on the interface plate 172 receives the working beams 180 in an orientation aligned with the motion axis 162 and redirects the working beams 180 in the direction of the motion axis 166 through an aperture 184 in the interface plate 170 to a reflector 186 that mounted together with the fast axis scanner 154 on the interface plate 170. The reflector 186 redirects the working beams 180 in the direction of the motion axis 158 above the fast axis scanner 154. Reflectors 188 and 190, which are also preferably mounted from the interface plate 170 redirect the working beams 180 within the same plane to a reflector 192, such as a fold prism, which aligns the working beams 180 with an optical axis 194 of the optics assembly 156.

Other types of single or multi-axis scanners can be incorporated, such as scanners using angularly scanning rotating polygon mirrors or angularly scanned galvanometer-controlled mirrors with image relaying systems to direct the working beams 180 over appropriate pathways for writing refractive structures 152 within an optical material.

The controllers and amplifiers 174 can also include a second synchronized arbitrary waveform generator for controlling a modulator 196, such as an electro-optic modulator or an acousto-optic modulator, for regulating the intensity of the working beams 180 in relation to motions along one or more of the motion axes 158, 162, and 166. For example, the beam intensity at the focal points 151 can be changed during a scan along the motion axis 15, or the beam intensity at the focal points can be reset to a new fixed value before each new trace is written. More integrated intensity control can be provided among the individual working beams including the distribution of pulse energy, as the working beams are moved along any combination of the motion axes 158, 162, and 166 to more fully regulate energy profiles within the optical material. The modulator 196, which can used to regulate the overall intensity or intensity distributions among the working beams is disposed in an optical path between a laser used for generating the working beams and a surface of the optical material.. However, the modulator 196 is preferably located in advance of the beamsplitters for variably attenuating multiple working beams. The controller 35 of FIGS. 1 and 7 can also be incorporated among the controllers and amplifiers 174 to adjust one or more of the half-wave plate 16 of FIGS. 1 and 7, the delay element 34 of FIG. 1, and the beam shaper of FIG. 7 for adjusting various parameters among the working beams 180.

Scanners such as the optomechanical scanner 150 can be arranged together with desired parameters for laser power, wavelength, and scan speed, to write millimeter-scale devices (up to about 8 mm wide) in the optical material at speeds exceeding 100 mm/sec. A lateral gradient index microlens can be written by changing the scanning speed after each trace is written, and/or by changing the laser intensity before the next trace is written. In addition, the index of refraction is changed by varying beam intensity or the scan speed along the length of a trace or by some combination of the two. Both positive lenses and negative lenses (as opposed to cylindrical lenses) can be written using a combination of overlapping lenses and synchronous intensity control. The overall refractive power can be tailored to the desired shape using these parameters, as well as global positioning and the laser modulator.

Further details of a useful scanning system are described in US Patent Application Publication No. 20160144580 A1 entitled HIGH NUMERICAL APERTURE OPTOMECHANICAL SCANNER FOR LAYERED GRADIENT INDEX MICROLENSES, METHODS, AND APPLICATIONS, Exemplary suitable methods and techniques have been described, for example, in U.S. Pat. No. 7,789,910 B2, OPTICAL MATERIAL AND METHOD FOR MODIFYING THE REFRACTIVE INDEX, to Knox, et. al.; U.S. Pat. No. 8,337,553 B2, OPTICAL MATERIAL AND METHOD FOR MODIFYING THE REFRACTIVE INDEX, to Knox, et. al.; U.S. Pat. No. 8,486,055 B2, METHOD FOR MODIFYING THE REFRACTIVE INDEX OF OCULAR TISSUES, to Knox, et. al.; U.S. Pat. No. 8,512,320 B1, METHOD FOR MODIFYING THE REFRACTIVE INDEX OF OCULAR TISSUES, to Knox, et. al.; and U.S. Pat. No. 8,617,147 B2, METHOD FOR MODIFYING THE REFRACTIVE INDEX OF OCULAR TISSUES.

The invention is defined in the following claims. Other embodiments, methods, examples etc. are provided for illustrative purposes only. In particular, in-vivo methods are not a part of the invention.

## Claims

1. A writing system comprising:
a pulsed laser source (12), an objective lens (38, 96, 126) for focusing an output (14) of the pulsed laser source (12) to a focal spot (40, 42; 72, 74; 100, 102; 130, 132; 130a, 132a; 130b, 132b) in an optical material (44), and a scanner (150) for relatively moving the focal spot (40, 42; 72, 74; 100, 102; 130, 132; 130a, 132a; 130b, 132b) with respect to the optical material (44) at a relative speed and direction along a scan region;
wherein
the optical material is an optical ophthalmic material (44);
the writing system is a refractive index writing system configured to write one or more traces (50, 52; 76, 78; 210, 212) in the optical ophthalmic material (44) defined by a change in refractive index (Δn);
the system being chracterised by further comprising:
a beam multiplexer (10, 60, 110, 140, 200) for dividing the output (14) of the laser source (12) into at least two working beams (20, 22; 62, 64; 90, 92; 120, 122; 120a, 122a; 120b, 122b; 180) that are focused by the objective lens (38, 96, 126) to focal spots (40, 42; 72, 74; 100, 102; 130, 132; 130a, 132a; 130b, 132b) that are at least one of temporally and spatially offset within the optical material (44); and
at least one controller (35) configured for controlling at least one of the temporal and spatial offset (Δs, Δv) between the focal spots (40, 42; 72, 74; 100, 102; 130, 132; 130a, 132a; 130b, 132b) of the working beams (20, 22; 62, 64; 90, 92; 120, 122; 120a, 122a; 120b, 122b; 180);
wherein
the scanner (150) is adjustable for setting the relative speed and direction of the focal spots (40, 42; 72, 74; 100, 102; 130, 132; 130a, 132a; 130b, 132b) for maintaining an energy profile within the optical ophthalmic material (44) along the scan region above a nonlinear absorption threshold of the optical ophthalmic material (44) and below a breakdown threshold of the optical ophthalmic material (44) at which significant light scattering or absorption degrades the intended performance of the optical ophthalmic material (44); and
the at least one controller (35) is also configured to regulate the scanner (150) for adjusting the relative speed and direction of the focal spots (40, 42; 72, 74; 100, 102; 130, 132; 130a, 132a; 130b, 132b) in the optical ophthalmic material (44) to maintain the energy profile below the breakdown threshold of the optical ophthalmic material (44).

2. The writing system of claim 1 in which the pulsed laser source (12) is arranged for producing a collimated output beam (14) composed of a succession of pulses having a pulse energy between 0.01 nJ and 10 nJ, a pulse duration between 8 fs and 500 fs, and a repetition rate between 10 MHz and 500 MHz.

3. The writing system of claim 2 in which the at least two working beams (20, 22; 62, 64; 90, 92; 120, 122; 120a, 122a; 120b, 122b; 180) as divided by the beam multiplexer (10, 60, 110, 140, 200) are composed of pulses sharing predetermined portions of the pulse energy of the output beam (14).

4. The writing system of any of claims 1 to 3 in which the beam multiplexer (10, 60, 110, 140, 200) is arranged for further distinguishing the at least two working beams (20, 22; 62, 64; 90, 92; 120, 122; 120a, 122a; 120b, 122b; 180) by at least one of a temporal offset (Δt), an angular offset (Δα), and a variation in beam width.

5. The writing system of any of claims 1 to 4 in which a beam shaper (66) reshapes at least one of the working beams (20, 22; 62, 64; 90, 92; 120, 122; 120a, 122a; 120b, 122b; 180) to elongate at least one of the focal spots (40, 42; 72, 74; 100, 102; 130, 132; 130a, 132a; 130b, 132b) in a direction of propagation.

6. The writing system of claim 4 in which the objective lens (38, 96, 126) is arranged for collectively focusing the working beams (20, 22; 62, 64; 90, 92; 120, 122; 120a, 122a; 120b, 122b; 180) to focal spots (40, 42; 72, 74; 100, 102; 130, 132; 130a, 132a; 130b, 132b) within the optical material (44).

7. The writing system of claim 6 in which the beam multiplexer (10, 60, 110, 140, 200) includes:
a first beamsplitter (18; 86; 116) that divides the output beam (14) into a first of the working beams (20, 22; 62, 64; 90, 92; 120, 122; 120a, 122a; 120b, 122b; 180) propagating along a first optical region and a second of the working beams (20, 22; 62, 64; 90, 92; 120, 122; 120a, 122a; 120b, 122b; 180) propagating along a second optical region, and
a second beamsplitter (28) that at least partially overlaps the first and second working beams (20, 22; 62, 64; 90, 92; 120, 122; 120a, 122a; 120b, 122b; 180) en route to the objective lens (38, 96, 126).

8. The writing system of claim 7 in which the first beamsplitter is a polarization beamsplitter (18) that propagates the first working beam (20; 62; 90; 120; 120a; 122a) with a first polarization and propagates the second working beam (22; 64; 92; 122; 122a; 122b) with a second polarization, and the beam multiplexer (10, 60, 110, 140, 200) includes a polarization rotator in advance of the first polarization beamsplitter (18) for adjusting the relative pulse energies of the first and second working beams (20, 22; 62, 64; 90, 92; 120, 122; 120a, 122a; 120b, 122b).

9. The writing system of claim 7 in which the first optical region is related to the second optical region for relatively angularly offsetting the first and second working beams (20, 22; 62, 64; 90, 92; 120, 122; 120a, 122a; 120b, 122b), and the objective lens (38, 96, 126) having an optical axis (39, 68, 95, 128, 144, 194) converts the angular offset (Δα) between the working beams (20, 22; 62, 64; 90, 92; 120, 122; 120a, 122a; 120b, 122b) into a spatial offset (Δs, Δv) between the focal spots (40, 42; 72, 74; 100, 102; 130, 132; 130a, 132a; 130b, 132b) in a given transverse plane along the optical axis (39, 68, 95, 128, 144, 194).

10. The writing system of claim 7 in which the beam multiplexer (10, 60, 110, 140, 200) includes an optic having optical power located along at least one of the first and second regions for imparting a progressive variation in a beam width of one of the first and second working beams (20, 22; 62, 64; 90, 92; 120, 122; 120a, 122a; 120b, 122b) with respect to the other of the first and second working beams (20, 22; 62, 64; 90, 92; 120, 122; 120a, 122a; 120b, 122b), and the objective lens (38, 96, 126) having an optical axis (39, 68, 95, 128, 144, 194) converts the progressive variation in beam width into a second spatial offset (Δs, Δv) between the focal spots (40, 42; 72, 74; 100, 102; 130, 132; 130a, 132a; 130b, 132b) in different transverse planes along the optical axis (39, 68, 95, 128, 144, 194).

11. The writing system of claim 1 further comprising a beam shaper (66) for elongating at least one of the focal spots (40, 42; 72, 74; 100, 102; 130, 132; 130a, 132a; 130b, 132b) in a direction of propagation.

12. The writing system of claim 11 in which the beam shaper (66) introduces spherical aberration into at least one of the working beams (20, 22; 62, 64; 90, 92; 120, 122; 120a, 122a; 120b, 122b; 180).

13. The writing system of claim 11 in which the at least one focal spot (40, 42; 72, 74; 100, 102; 130, 132; 130a, 132a; 130b, 132b) is elongated in the direction of propagation by an amount that is capable of supporting a 2π phase change for a nominal wavelength intended for propagation through the optical ophthalmic material (44).

14. The writing system of claim 11 in which the focal spots (40, 42; 72, 74; 100, 102; 130, 132; 130a, 132a; 130b, 132b) of the two working beams (20, 22; 62, 64; 90, 92; 120, 122; 120a, 122a; 120b, 122b; 180) are offset in the direction of propagation and are collectively elongated in the direction of propagation by an amount that is capable of supporting a 2π phase change for a nominal wavelength intended for propagation through the optical ophthalmic material (44).

15. A method of ex vivo writing localized refractive index changes (Δn) in optical ophthalmic materials (44) within an energy regimes above a nonlinear absorption threshold of the optical ophthalmic materials (44) and below a breakdown threshold of the optical ophthalmic materials (44) at which significant light scattering or absorption degrades their intended performance using a writing system of any one of claims 1 to 14, wherein the optical ophthalmic material (44) is synthetically produced ophthalmic material (44), the method comprising steps of:
producing a collimated output beam (14) composed of a succession of pulses having a pulse energy between 0.01 nJ and 10 nJ, a pulse duration between 8 fs and 500 fs, and a repetition rate between 10 MHz and 500 MHz;
dividing the collimated output beam (14) into at least two working beams composed of pulses sharing predetermined portions of the pulse energy of the output beam (14);
collectively focusing the working beams with an objective lens (38, 96, 126) to different focal spots (40, 42; 72, 74; 100, 102; 130, 132; 130a, 132a; 130b, 132b) within the optical ophthalmic material (44) at which at least one of:
the different focal spots (40, 42; 72, 74; 100, 102; 130, 132; 130a, 132a; 130b, 132b) are focused at a first spatial offset (Δs, Δv) in a given plane, and
the different focal spots (40, 42; 72, 74; 100, 102; 130, 132; 130a, 132a; 130b, 132b) are focused at a second spatial offset (Δs, Δv) in different planes at different distances from the objective lens (38, 96, 126); and
relatively moving the objective lens (38, 96, 126) with respect to the optical ophthalmic material (44) at a relative speed and relative direction at which the different focal spots (40, 42; 72, 74; 100, 102; 130, 132; 130a, 132a; 130b, 132b) write a series of traces (50, 52; 76, 78; 210, 212) defined by a change in refractive index (Δn) of the optical ophthalmic material (44) for achieving a desired refractive index profile in the optical ophthalmic material (44).

## Patentansprüche

1. Schreibsystem, umfassend:
eine gepulste Laserquelle (12), eine Objektivlinse (38, 96, 126) zum Fokussieren eines Ausgangs (14) der gepulsten Laserquelle (12) auf einen Brennpunkt (40, 42; 72, 74; 100, 102; 130, 132; 130a, 132a; 130b, 132b) in einem optischen Material (44), und einen Scanner (150) zum relativen Bewegen des Brennpunkts (40, 42; 72, 74; 100, 102; 130, 132; 130a, 132a; 130b, 132b) in Bezug auf das optische Material (44) mit einer relativen Geschwindigkeit und Richtung entlang eines Scanbereichs;
wobei
das optische Material ein optisches ophthalmisches Material (44) ist;
das Schreibsystem ein Brechungsindex-Schreibsystem ist, das so ausgestaltet ist, dass es eine oder mehrere Spuren (50, 52; 76, 78; 210, 212) in das optische ophthalmische Material (44) schreibt, die durch eine Änderung des Brechungsindex (Δn) definiert sind;
wobei das System **dadurch gekennzeichnet ist, dass** es ferner Folgendes umfasst:
einen Strahlmultiplexer (10, 60, 110, 140, 200) zum Aufteilen des Ausgangs (14) der Laserquelle (12) in mindestens zwei Arbeitsstrahlen (20, 22; 62, 64; 90, 92; 120, 122; 120a, 122a; 120b, 122b; 180), die durch die Objektivlinse (38, 96, 126) auf Brennpunkte (40, 42; 72, 74; 100, 102; 130, 132; 130a, 132a; 130b, 132b) fokussiert werden, die innerhalb des optischen Materials (44) zumindest zeitlich oder räumlich versetzt sind; und
mindestens ein Steuergerät (35), das so ausgestaltet ist, dass es mindestens einen der zeitlichen und räumlichen Versätze (Δs, Δv) zwischen den Brennpunkten (40, 42; 72, 74; 100, 102; 130, 132; 130a, 132a; 130b, 132b) der Arbeitsstrahlen (20, 22; 62, 64; 90, 92; 120, 122; 120a, 122a; 120b, 122b; 180) steuert;
wobei
der Scanner (150) einstellbar ist, um die relative Geschwindigkeit und Richtung der Brennpunkte (40, 42; 72, 74; 100, 102; 130, 132; 130a, 132a; 130b, 132b) zum Aufrechterhalten eines Energieprofils innerhalb des optischen ophthalmischen Materials (44) entlang des Scanbereichs oberhalb eines nichtlinearen Absorptionsschwellenwerts des optischen ophthalmischen Materials (44) und unterhalb eines Durchbruchschwellenwerts des optischen ophthalmischen Materials (44), bei dem eine signifikante Lichtstreuung oder -absorption die beabsichtigte Leistung des optischen ophthalmischen Materials (44) verschlechtert, einzustellen; und
das mindestens eine Steuergerät (35) auch so ausgestaltet ist, dass es den Scanner (150) zum Einstellen der relativen Geschwindigkeit und Richtung der Brennpunkte (40, 42; 72, 74; 100, 102; 130, 132; 130a, 132a; 130b, 132b) in dem optischen ophthalmischen Material (44) reguliert, um das Energieprofil unter der Durchbruchschwelle des optischen ophthalmischen Materials (44) zu halten.

2. Schreibsystem nach Anspruch 1, bei dem die gepulste Laserquelle (12) so angeordnet ist, dass sie einen kollimierten Ausgangsstrahl (14) erzeugt, der aus einer Folge von Pulsen mit einer Pulsenergie zwischen 0,01 nJ und 10 nJ, einer Pulsdauer zwischen 8 fs und 500 fs und einer Wiederholungsrate zwischen 10 MHz und 500 MHz besteht.

3. Schreibsystem nach Anspruch 2, bei dem die mindestens zwei Arbeitsstrahlen (20, 22; 62, 64; 90, 92; 120, 122; 120a, 122a; 120b, 122b; 180), wie sie vom Strahlenmultiplexer (10, 60, 110, 140, 200) geteilt werden, aus Pulsen bestehen, die sich vorbestimmte Teile der Pulsenergie des Ausgangsstrahls (14) teilen.

4. Schreibsystem nach einem der Ansprüche 1 bis 3, bei dem der Strahlenmultiplexer (10, 60, 110, 140, 200) so eingerichtet ist, dass er die mindestens zwei Arbeitsstrahlen (20, 22; 62, 64; 90, 92; 120, 122; 120a, 122a; 120b, 122b; 180) durch mindestens einen zeitlichen Versatz (Δt), einen Winkelversatz (Δα) oder eine Veränderung der Strahlbreite weiter unterscheidet.

5. Schreibsystem nach einem der Ansprüche 1 bis 4, bei dem ein Strahlformer (66) mindestens einen der Arbeitsstrahlen (20, 22; 62, 64; 90, 92; 120, 122; 120a, 122a; 120b, 122b; 180) umformt, um mindestens einen der Brennpunkte (40, 42; 72, 74; 100, 102; 130, 132; 130a, 132a; 130b, 132b) in einer Ausbreitungsrichtung zu verlängern.

6. Schreibsystem nach Anspruch 4, bei dem die Objektivlinse (38, 96, 126) zur kollektiven Fokussierung der Arbeitsstrahlen (20, 22; 62, 64; 90, 92; 120, 122; 120a, 122a; 120b, 122b; 180) auf Brennpunkte (40, 42; 72, 74; 100, 102; 130, 132; 130a, 132a; 130b, 132b) innerhalb des optischen Materials (44) angeordnet ist.

7. Schreibsystem nach Anspruch 6, bei dem der Strahlmultiplexer (10, 60, 110, 140, 200) umfasst:
einen ersten Strahlteiler (18; 86; 116), der den Ausgangsstrahl (14) in einen ersten der Arbeitsstrahlen (20, 22; 62, 64; 90, 92; 120, 122; 120a, 122a; 120b, 122b; 180), der sich entlang eines ersten optischen Bereichs ausbreitet, und einen zweiten der Arbeitsstrahlen (20, 22; 62, 64; 90, 92; 120, 122; 120a, 122a; 120b, 122b; 180), der sich entlang eines zweiten optischen Bereichs ausbreitet, teilt, und
einen zweiten Strahlenteiler (28), der den ersten und den zweiten Arbeitsstrahl (20, 22; 62, 64; 90, 92; 120, 122; 120a, 122a; 120b, 122b; 180) auf dem Weg zur Objektivlinse (38, 96, 126) zumindest teilweise überlappt.

8. Schreibsystem nach Anspruch 7, bei dem der erste Strahlenteiler ein Polarisationsstrahlenteiler (18) ist, der den ersten Arbeitsstrahl (20; 62; 90; 120; 120a; 122a) mit einer ersten Polarisation ausbreitet und den zweiten Arbeitsstrahl (22; 64; 92; 122; 122a; 122b) mit einer zweiten Polarisation ausbreitet, und der Strahlmultiplexer (10, 60, 110, 140, 200) einen Polarisationsrotator vor dem ersten Polarisations-Strahlteiler (18) enthält, um die relativen Pulsenergien des ersten und zweiten Arbeitsstrahls (20, 22; 62, 64; 90, 92; 120, 122; 120a, 122a; 120b, 122b) einzustellen.

9. Schreibsystem nach Anspruch 7, bei dem der erste optische Bereich mit dem zweiten optischen Bereich in Beziehung steht, um den ersten und den zweiten Arbeitsstrahl (20, 22; 62, 64; 90, 92; 120, 122; 120a, 122a; 120b, 122b) relativ winkelmäßig zu versetzen, und die Objektivlinse (38, 96, 126), dieeine optische Achse (39, 68, 95, 128, 144, 194) hat, den winkelmäßigen Versatz (Δα) zwischen den Arbeitsstrahlen (20, 22; 62, 64; 90, 92; 120, 122; 120a, 122a; 120b, 122b) in einen räumlichen Versatz (Δs, Δv) zwischen den Brennpunkten (40, 42; 72, 74; 100, 102; 130, 132; 130a, 132a; 130b, 132b) in einer gegebenen Querebene entlang der optischen Achse (39, 68, 95, 128, 144, 194) umwandelt.

10. Schreibsystem nach Anspruch 7, bei dem der Strahlenmultiplexer (10, 60, 110, 140, 200) eine Optik mit optischer Leistung enthält, die entlang mindestens eines der ersten und zweiten Bereiche angeordnet ist, um eine progressive Veränderung der Strahlbreite eines der ersten und zweiten Arbeitsstrahlen (20, 22; 62, 64; 90, 92; 120, 122; 120a, 122a; 120b, 122b) in Bezug auf den anderen der ersten und zweiten Arbeitsstrahlen (20, 22; 62, 64; 90, 92; 120, 122; 120a, 122a; 120b, 122b) zu übermitteln, und die Objektivlinse (38, 96, 126), die eine optische Achse (39, 68, 95, 128, 144, 194) hat, die progressive Veränderung der Strahlbreite in einen zweiten räumlichen Versatz (Δs, Δv) zwischen den Brennpunkten (40, 42; 72, 74; 100, 102; 130, 132; 130a, 132a; 130b, 132b) in verschiedenen Querebenen entlang der optischen Achse (39, 68, 95, 128, 144, 194) umwandelt.

11. Schreibsystem nach Anspruch 1, ferner umfassend einen Strahlformer (66) zum Verlängern mindestens eines der Brennpunkte (40, 42; 72, 74; 100, 102; 130, 132; 130a, 132a; 130b, 132b) in einer Ausbreitungsrichtung.

12. Schreibsystem nach Anspruch 11, bei dem der Strahlformer (66) eine sphärische Abweichung in mindestens einen der Arbeitsstrahlen (20, 22; 62, 64; 90, 92; 120, 122; 120a, 122a; 120b, 122b; 180) einführt.

13. Schreibsystem nach Anspruch 11, bei dem der mindestens eine Brennpunkt (40, 42; 72, 74; 100, 102; 130, 132; 130a, 132a; 130b, 132b) in Ausbreitungsrichtung um einen Betrag verlängert ist, der geeignet ist, eine 2π-Phasenänderung für eine nominale Wellenlänge zu unterstützen, die für die Ausbreitung durch das optische ophthalmische Material (44) vorgesehen ist.

14. Schreibsystem nach Anspruch 11, bei dem die Brennpunkte (40, 42; 72, 74; 100, 102; 130, 132; 130a, 132a; 130b, 132b) von den beiden Arbeitsstrahlen (20, 22; 62, 64; 90, 92; 120, 122; 120a, 122a; 120b, 122b; 180) in der Ausbreitungsrichtung versetzt sind und gemeinsam in der Ausbreitungsrichtung um einen Betrag verlängert sind, der geeignet ist, eine 2π-Phasenänderung für eine nominale Wellenlänge zu unterstützen, die für die Ausbreitung durch das optische ophthalmische Material (44) vorgesehen ist.

15. Verfahren zum Ex-vivo-Schreiben von lokalisierten Brechungsindexänderungen (Δn) in optische ophthalmische Materialien (44) innerhalb eines Energiebereichs oberhalb eines nichtlinearen Absorptionsschwellenwerts der optischen ophthalmischen Materialien (44) und unterhalb eines Durchbruchschwellenwerts der optischen ophthalmischen Materialien (44), bei dem eine signifikante Lichtstreuung oder -absorption ihre beabsichtigte Leistung beeinträchtigt, unter Verwendung eines Schreibsystems nach einem der Ansprüche 1 bis 14, wobei das optische ophthalmische Material (44) ein synthetisch hergestelltes ophthalmisches Material (44) ist, wobei das Verfahren die folgenden Schritte umfasst:
Erzeugung eines kollimierten Ausgangsstrahls (14), der aus einer Folge von Pulsen mit einer Pulsenergie zwischen 0,01 nJ und 10 nJ, einer Pulsdauer zwischen 8 fs und 500 fs und einer Wiederholungsrate zwischen 10 MHz und 500 MHz besteht;
Aufteilung des kollimierten Ausgangsstrahls (14) in mindestens zwei Arbeitsstrahlen, die sich aus Pulsen zusammensetzen, die sich vorbestimmte Teile der Pulsenergie des Ausgangsstrahls (14) teilen;
kollektives Fokussieren der Arbeitsstrahlen mit einer Objektivlinse (38, 96, 126) auf verschiedene Brennpunkte (40, 42; 72, 74; 100, 102; 130, 132; 130a, 132a; 130b, 132b) innerhalb des optischen ophthalmischen Materials (44), an denen mindestens einer der folgenden Punkte liegt:
die verschiedenen Brennpunkte (40, 42; 72, 74; 100, 102; 130, 132; 130a, 132a; 130b, 132b) werden in einer bestimmten Ebene mit einem ersten räumlichen Versatz (Δs, Δv) fokussiert, und
die verschiedenen Brennpunkte (40, 42; 72, 74; 100, 102; 130, 132; 130a, 132a; 130b, 132b) werden mit einem zweiten räumlichen Versatz (Δs, Δv) in verschiedenen Ebenen in unterschiedlichen Abständen von der Objektivlinse (38, 96, 126) fokussiert; und
relatives Bewegen der Objektivlinse (38, 96, 126) in Bezug auf das optische ophthalmische Material (44) mit einer relativen Geschwindigkeit und einer relativen Richtung, bei der die verschiedenen Brennpunkte (40, 42; 72, 74; 100, 102; 130, 132; 130a, 132a; 130b, 132b) eine Reihe von Spuren (50, 52; 76, 78; 210, 212) schreiben, die durch eine Änderung des Brechungsindex (Δn) des optischen ophthalmischen Materials (44) definiert sind, um ein gewünschtes Brechungsindexprofil in dem optischen ophthalmischen Material (44) zu erreichen.

## Revendications

1. Un système d'écriture comprenant:
une source laser pulsée (12), une lentille d'objectif (38, 96, 126) pour focaliser une sortie (14) de la source laser pulsée (12) sur un point focal (40, 42; 72, 74; 100, 102; 130, 132; 130a, 132a; 130b, 132b) dans un matériau optique (44), et un scanner (150) pour déplacer relativement le point focal (40, 42; 72, 74; 100, 102; 130, 132; 130a, 132a; 130b, 132b) par rapport au matériau optique (44) à une vitesse et une direction relatives le long d'une région de balayage;
où
le matériau optique est un matériau optique ophtalmique (44);
le système d'écriture est un système d'écriture d'indice de réfraction configuré pour écrire une ou plusieurs traces (50, 52; 76, 78; 210, 212) dans le matériau optique ophtalmique (44) définies par un changement d'indice de réfraction (Δn);
le système étant **caractérisé par le fait qu'**il comprend en outre:
un multiplexeur de faisceau (10, 60, 110, 140, 200) pour diviser la sortie (14) de la source laser (12) en au moins deux faisceaux de travail (20, 22; 62, 64; 90, 92; 120, 122; 120a, 122a; 120b, 122b; 180) qui sont focalisés par la lentille d'objectif (38, 96, 126) vers des points focaux (40, 42; 72, 74; 100, 102; 130, 132; 130a, 132a; 130b, 132b) qui sont au moins l'un parmi un décalage temporel et spatial dans le matériau optique (44); et
au moins un contrôleur (35) configuré pour contrôler au moins l'un des décalages temporel et spatial (Δs, Δv) entre les points focaux (40, 42; 72, 74; 100, 102; 130, 132; 130a, 132a; 130b, 132b) des faisceaux de travail (20, 22; 62, 64; 90, 92; 120, 122; 120a, 122a; 120b, 122b; 180);
où
le scanner (150) est réglable pour régler la vitesse et la direction relatives des points focaux (40, 42; 72, 74; 100, 102; 130, 132; 130a, 132a; 130b, 132b) pour maintenir un profil d'énergie à l'intérieur du matériau ophtalmique optique (44) le long de la région de balayage au-dessus d'un seuil d'absorption non linéaire du matériau ophtalmique optique (44) et au-dessous d'un seuil de rupture du matériau ophtalmique optique (44) auquel une diffusion ou une absorption significative de la lumière dégrade les performances prévues du matériau ophtalmique optique (44); et
l'au moins un contrôleur (35) est également configuré pour réguler le scanner (150) afin d'ajuster la vitesse et la direction relatives des points focaux (40, 42; 72, 74; 100, 102; 130, 132; 130a, 132a; 130b, 132b) dans le matériau ophtalmique optique (44) pour maintenir le profil énergétique en dessous du seuil de rupture du matériau ophtalmique optique (44).

2. Système d'écriture selon la revendication 1, dans lequel la source laser pulsée (12) est conçue pour produire un faisceau de sortie collimaté (14) composé d'une séquence de pulsations ayant une énergie pulsée comprise entre 0,01 nJ et 10 nJ, une durée de pulsation comprise entre 8 fs et 500 fs, et une fréquence de répétition comprise entre 10 MHz et 500 MHz.

3. Système d'écriture selon la revendication 2, dans lequel les au moins deux faisceaux de travail (20, 22; 62, 64; 90, 92; 120, 122; 120a, 122a; 120b, 122b; 180) tels que divisés par le multiplexeur de faisceaux (10, 60, 110, 140, 200) sont composés de pulsations partageant des parties prédéterminées de l'énergie pulsée du faisceau de sortie (14).

4. Système d'écriture de l'une quelconque des revendications 1 à 3, dans lequel le multiplexeur de faisceau (10, 60, 110, 140, 200) est agencé pour distinguer en outre les au moins deux faisceaux de travail (20, 22; 62, 64; 90, 92; 120, 122; 120a, 122a; 120b, 122b; 180) par au moins l'un d'un décalage temporel (Δt), d'un décalage angulaire (Δα) et d'une variation de largeur de faisceau.

5. Système d'écriture de l'une quelconque des revendications 1 à 4, dans lequel un dispositif de mise en forme de faisceau (66) remet en forme au moins l'un des faisceaux de travail (20, 22; 62, 64; 90, 92; 120, 122; 120a, 122a; 120b, 122b; 180) pour allonger au moins l'un des points focaux (40, 42; 72, 74; 100, 102; 130, 132; 130a, 132a; 130b, 132b) dans une direction de propagation.

6. Système d'écriture selon la revendication 4, dans lequel la lentille d'objectif (38, 96, 126) est agencée pour focaliser collectivement les faisceaux de travail (20, 22; 62, 64; 90, 92; 120, 122; 120a, 122a; 120b, 122b; 180) vers des points focaux (40, 42; 72, 74; 100, 102; 130, 132; 130a, 132a; 130b, 132b) dans le matériau optique (44).

7. Système d'écriture selon la revendication 6, dans lequel le multiplexeur de faisceau (10, 60, 110, 140, 200) comprend:
un premier diviseur de faisceau (18; 86; 116) qui divise le faisceau de sortie (14) en un premier des faisceaux de travail (20, 22; 62, 64; 90, 92; 120, 122; 120a, 122a; 120b, 122b; 180) se propageant le long d'une première région optique et un second des faisceaux de travail (20, 22; 62, 64; 90, 92; 120, 122; 120a, 122a; 120b, 122b; 180) se propageant le long d'une seconde région optique, et
un second diviseur de faisceau (28) qui chevauche au moins partiellement les premier et second faisceaux de travail (20, 22; 62, 64; 90, 92; 120, 122; 120a, 122a; 120b, 122b; 180) en route vers la lentille d'objectif (38, 96, 126).

8. Système d'écriture selon la revendication 7, dans lequel le premier diviseur de faisceau est un diviseur de faisceau à polarisation (18) qui propage le premier faisceau de travail (20; 62; 90; 120; 120a; 122a) avec une première polarisation et propage le second faisceau de travail (22; 64; 92; 122; 122a; 122b) avec une seconde polarisation, et le multiplexeur de faisceau (10, 60, 110, 140, 200) comprend un rotateur de polarisation en amont du diviseur de faisceau à première polarisation (18) pour ajuster les énergies pulsée relatives des premier et second faisceaux de travail (20, 22; 62, 64; 90, 92; 120, 122; 120a, 122a; 120b, 122b).

9. Système d'écriture selon la revendication 7, dans lequel la première région optique est liée à la seconde région optique pour décaler angulairement de manière relative les premier et second faisceaux de travail (20, 22; 62, 64; 90, 92; 120, 122; 120a, 122a; 120b, 122b), et la lentille d'objectif (38, 96, 126) ayant un axe optique (39, 68, 95, 128, 144, 194) convertit le décalage angulaire (Δα) entre les faisceaux de travail (20, 22; 62, 64; 90, 92; 120, 122; 120a, 122a; 120b, 122b) en un décalage spatial (Δs, Δv) entre les points focaux (40, 42; 72, 74; 100, 102; 130, 132; 130a, 132a; 130b, 132b) dans un plan transversal donné le long de l'axe optique (39, 68, 95, 128, 144, 194).

10. Système d'écriture selon la revendication 7, dans lequel le multiplexeur de faisceau (10, 60, 110, 140, 200) comprend une optique ayant une puissance optique située le long d'au moins l'une des première et deuxième régions pour communiquer une variation progressive de la largeur de faisceau de l'un des premier et deuxième faisceaux de travail (20, 22; 62, 64; 90, 92; 120, 122; 120a, 122a; 120b, 122b) par rapport à l'autre des premier et deuxième faisceaux de travail (20, 22; 62, 64; 90, 92; 120, 122; 120a, 122a; 120b, 122b), et la lentille d'objectif (38, 96, 126) ayant un axe optique (39, 68, 95, 128, 144, 194) convertit la variation progressive de la largeur du faisceau en un second décalage spatial (Δs, Δv) entre les points focaux (40, 42; 72, 74; 100, 102; 130, 132; 130a, 132a; 130b, 132b) dans différents plans transversaux le long de l'axe optique (39, 68, 95, 128, 144, 194).

11. Système d'écriture de la revendication 1 comprenant en outre un dispositif de mise en forme de faisceau (66) pour allonger au moins l'un des points focaux (40, 42; 72, 74; 100, 102; 130, 132; 130a, 132a; 130b, 132b) dans une direction de propagation.

12. Système d'écriture selon la revendication 11, dans lequel le conformateur de faisceau (66) introduit une aberration sphérique dans au moins un des faisceaux de travail (20, 22; 62, 64; 90, 92; 120, 122; 120a, 122a; 120b, 122b; 180).

13. Système d'écriture selon la revendication 11, dans lequel au moins un point focal (40, 42; 72, 74; 100, 102; 130, 132; 130a, 132a; 130b, 132b) est allongée dans la direction de propagation d'une quantité qui est capable de supporter un changement de phase de 2π pour une longueur d'onde nominale destinée à la propagation à travers le matériau optique ophtalmique (44).

14. Système d'écriture selon la revendication 11, dans lequel les points focaux (40, 42; 72, 74; 100, 102; 130, 132; 130a, 132a; 130b, 132b) de les deux faisceaux de travail (20, 22; 62, 64; 90, 92; 120, 122; 120a, 122a; 120b, 122b; 180) sont décalés dans la direction de propagation et sont collectivement allongés dans la direction de propagation d'une quantité qui est capable de supporter un changement de phase de 2π pour une longueur d'onde nominale destinée à la propagation à travers le matériau optique ophtalmique (44).

15. Procédé d'écriture ex vivo de changements d'indice de réfraction localisés (Δn) dans des matériaux ophtalmiques optiques (44) dans des régimes d'énergie supérieurs à un seuil d'absorption non linéaire des matériaux ophtalmiques optiques (44) et inférieurs à un seuil de rupture des matériaux ophtalmiques optiques (44) auquel une diffusion ou une absorption significative de la lumière dégrade leurs performances prévues en utilisant un système d'écriture de l'une quelconque des revendications 1 à 14, dans lequel le matériau ophtalmique optique (44) est un matériau ophtalmique produit synthétiquement (44), le procédé comprenant les étapes consistant à:
produire un faisceau de sortie collimaté (14) composé d'une séquence de pulsations ayant une énergie pulsée comprise entre 0,01 nJ et 10 nJ, une durée des pulsations comprise entre 8 fs et 500 fs, et une fréquence de répétition comprise entre 10 MHz et 500 MHz;
diviser le faisceau de sortie collimaté (14) en au moins deux faisceaux de travail composés de pulsations partageant des parties prédéterminées de l'énergie d'impulsion du faisceau de sortie (14);
focaliser collectivement les faisceaux de travail avec une lentille d'objectif (38, 96, 126) vers différents points focaux (40, 42; 72, 74; 100, 102; 130, 132; 130a, 132a; 130b, 132b) à l'intérieur du matériau optique ophtalmique (44) auquel au moins l'un de:
les différentes point focaux (40, 42; 72, 74; 100, 102; 130, 132; 130a, 132a; 130b, 132b) sont focalisées à un premier décalage spatial (Δs, Δv) dans un plan donné, et
les différentes points focaux (40, 42; 72, 74; 100, 102; 130, 132; 130a, 132a; 130b, 132b) sont focalisées à un second décalage spatial (Δs, Δv) dans différents plans à différentes distances de la lentille d'objectif (38, 96, 126); et
le déplacement relatif de la lentille d'objectif (38, 96, 126) par rapport au matériau optique ophtalmique (44) à une vitesse relative et dans une direction relative auxquelles les différents point focaux (40, 42; 72, 74; 100, 102; 130, 132; 130a, 132a; 130b, 132b) écrivent une série de traces (50, 52; 76, 78; 210, 212) définies par un changement d'indice de réfraction (Δn) du matériau ophtalmique optique (44) pour obtenir un profil d'indice de réfraction souhaité dans le matériau ophtalmique optique (44).
